# EUROPEAN PATENT SPECIFICATION

(11) **EP 1 345 495 B1**
(45) Date of publication and mention of the grant of the patent: **25.02.2009**
(21) Application number: 00977014.0
(22) Date of filing: 07.11.2000
(51) Int. Cl.: A01N 63/00, A61K 39/395, A61K 48/00, C07H 21/04, C12N 5/00, C12N 15/00, C12P 21/06

(54) **METHODS FOR GENERATING GENETICALLY ALTERED ANTIBODY-PRODUCING CELL LINES WITH IMPROVED ANTIBODY CHARACTERISTICS**
VERFAHREN ZUR HERSTELLUNG VON GENETISCH MODIFIZIERTEN ANTIKÖRPERPRODUZIERENDEN ZELLLINIEN MIT VERBESSERTEN ANTIKÖRPEREIGENSCHAFTEN
PROCEDES POUR GENERER DES LIGNEES CELLULAIRES PRODUISANT DES ANTICORPS GENETIQUEMENT MODIFIES PRESENTANT DES CARACTERISTIQUES AMELIOREES

(43) Date of publication of application: 24.09.2003
(60) Divisional application: 08167325.3
(73) Proprietor: Morphotek, Inc., Exton, PA 19341 (US)
(72) Inventor: NICOLAIDES, Nicholas, C., Garnet Valley Pennsylvania PA 19061 (US); GRASSO, Luigi, Bryn Mawr Pennsylvania PA 19010 (US); SASS, Philip, M., Audubon, PA 19403 (US)
(74) Representative: W.P. Thompson & Co.
(86) International application number: PCT/US2000/030588
(87) International publication number: WO 2002/037967

(56) References cited:
- CA-A1- 2 240 609
- IRVING R A ET AL: "Affinity maturation of recombinant antibodies using E.coli mutator cells" IMMUNOTECHNOLOGY, ELSEVIER SCIENCE PUBLISHERS BV, NL, vol. 2, no. 2, June 1996 (1996-06), pages 127-143, XP004052677 ISSN: 1380-2933
- NICOLAIDES NICHOLAS C ET AL: "A naturally occurring hPMS2 mutation can confer a dominant negative mutator phenotype" MOLECULAR AND CELLULAR BIOLOGY, AMERICAN SOCIETY FOR MICROBIOLOGY, WASHINGTON, US, vol. 18, no. 3, March 1998 (1998-03), pages 1635-1641, XP002165242 ISSN: 0270-7306
- WIESENDANGER MARGRIT ET AL: "Somatic hypermutation, transcription, and DNA mismatch repair" CELL, vol. 94, no. 4, 21 August 1998 (1998-08-21), pages 415-418, XP002333265 ISSN: 0092-8674
- REYNAUD C-A ET AL: "Mismatch repair and immunoglobulin gene hypermutation: did we learn something?" IMMUNOLOGY TODAY, ELSEVIER PUBLICATIONS, CAMBRIDGE, GB, vol. 20, no. 11, November 1999 (1999-11), pages 522-527, XP004183761 ISSN: 0167-5699
- KONG Q. ET AL.: 'PMS2-deficiency deminishes hypermutation of a lambdal transgene in young but not older mice' MOLECULAR IMMUNOLOGY vol. 36, 1999, pages 83 - 91, XP002937996
- VORA K.A. ET AL.: 'Severe attenuation of the B cell immune response in Msh2-deficient mice' JOURNAL OF EXPERIMENTAL MEDICINE vol. 189, no. 3, February 1999, pages 471 - 481, XP002937997
- WINTER D.B. ET AL.: 'Altered spectra of hypermulation in antibodies from mice deficient for the DNA mismatch repair protein PMS2' PROC. NATL. ACAD. SCI. USA vol. 95, June 1998, pages 6953 - 6958, XP002937998
- SCHRADER C.E. ET AL.: 'Reduced isotype switching in splenic B cells from mice deficient in mismatch repair enzymes' JOURNAL OF EXPERIMENTAL MEDICINE vol. 190, no. 3, 1999, pages 323 - 330, XP002937999

### SEQUENCE LISTING

<110> Morphotek Inc.
   Nicolaides, Nicholas C.
   Grasso, Luigi
   Sass, Philip M.
<120> METHODS FOR GENERATING GENETICALLY ALTERED ANTIBODY-PRODUCING CELL LINES WITH IMPROVED ANTIBODY CHARACTERISTICS
<130> MOR-0152
<150> PCT/US00/30588
   <151> 2000-11-07
<160> 24
<170> PatentIn version 3.3
<210> 1
   <211> 24
   <212> DNA
   <213> Artificial
<220>
   <223> Oligonucleotide primer
<400> 1
   ggattttcag gtgcagattt tcag
   24
<210> 2
   <211> 21
   <212> DNA
   <213> Artificial
<220>
   <223> Oligonucleotide primer
<400> 2
   actggatggt gggaagatgg a
   21
<210> 3
   <211> 19
   <212> DNA
   <213> Artificial
<220>
   <223> Oligonucleotide primer
<220>
   <221> misc_feature
   <222> (5)..(5)
   <223> n is a, c, g, or t
<220>
   <221> misc_feature
   <222> (11)..(11)
   <223> n is a, c, g, or t
<400> 3
   akgtnmagct ncagsagtc
   19
<210> 4
   <211> 19
   <212> DNA
   <213> Artificial
<220>
   <223> Oligonucleotide primer
<220>
   <221> misc_feature
   <222> (2)..(2)
   <223> n is a, c, g, or t
<400> 4
   tnccttgrcc ccagtarwc
   19
<210> 5
   <211> 859
   <212> PRT
   <213> Mus musculus
<400> 5
   Met Glu Gln Thr Glu Gly Val Ser Thr Glu Cys Ala Lys Ala Ile Lys
   1 5 10 15
   Pro Ile Asp Gly Lys Ser Val His Gln Ile Cys Ser Gly Gln Val Ile
   20 25 30
   Leu Ser Leu Ser Thr Ala Val Lys Glu Leu Ile Glu Asn Ser Val Asp
   35 40 45
   Ala Gly Ala Thr Thr Ile Asp Leu Arg Leu Lys Asp Tyr Gly Val Asp
   50 55 60
   Leu Ile Glu Val Ser Asp Asn Gly Cys Gly Val Glu Glu Glu Asn Phe
   65 70 75 80
   Glu Gly Leu Ala Leu Lys His His Thr Ser Lys Ile Gln Glu Phe Ala
   85 90 95
   Asp Leu Thr Gln Val Glu Thr Phe Gly Phe Arg Gly Glu Ala Leu Ser
   100 105 110
   Ser Leu Cys Ala Leu Ser Asp Val Thr Ile Ser Thr Cys His Gly Ser
   115 120 125
   Ala Ser Val Gly Thr Arg Leu Val Phe Asp His Asn Gly Lys Ile Thr
   130 135 140
   Gln Lys Thr Pro Tyr Pro Arg Pro Lys Gly Thr Thr Val Ser Val Gln
   145                 150                 155                 160
   His Leu Phe Tyr Thr Leu Pro Val Arg Tyr Lys Glu Phe Gln Arg Asn
                   165                 170                 175
   Ile Lys Lys Glu Tyr Ser Lys Met Val Gln Val Leu Gln Ala Tyr Cys
               180                 185                 190
   Ile Ile Ser Ala Gly Val Arg Val Ser Cys Thr Asn Gln Leu Gly Gln
           195                 200                 205
   Gly Lys Arg His Ala Val Val Cys Thr Ser Gly Thr Ser Gly Met Lys
       210                 215                 220
   Glu Asn Ile Gly Ser Val Phe Gly Gln Lys Gln Leu Gln Ser Leu Ile
   225                 230                 235                 240
   Pro Phe Val Gln Leu Pro Pro Ser Asp Ala Val Cys Glu Glu Tyr Gly
                   245                 250                 255
   Leu Ser Thr Ser Gly Arg His Lys Thr Phe Ser Thr Phe Arg Ala Ser
               260                 265                 270
   Phe His Ser Ala Arg Thr Ala Pro Gly Gly Val Gln Gln Thr Gly Ser
           275                 280                 285
   Phe Ser Ser Ser Ile Arg Gly Pro Val Thr Gln Gln Arg Ser Leu Ser
       290                 295                 300
   Leu Ser Met Arg Phe Tyr His Met Tyr Asn Arg His Gln Tyr Pro Phe
   305                 310                 315                 320
   Val Val Leu Asn Val Ser Val Asp Ser Glu Cys Val Asp Ile Asn Val
                   325                 330                 335
   Thr Pro Asp Lys Arg Gln Ile Leu Leu Gln Glu Glu Lys Leu Leu Leu
               340                 345                 350
   Ala Val Leu Lys Thr Ser Leu Ile Gly Met Phe Asp Ser Asp Ala Asn
           355                 360                 365
   Lys Leu Asn Val Asn Gln Gln Pro Leu Leu Asp Val Glu Gly Asn Leu
       370                 375                 380
   Val Lys Leu His Thr Ala Glu Leu Glu Lys Pro Val Pro Gly Lys Gln
   385                 390                 395                 400
   Asp Asn Ser Pro Ser Leu Lys Ser Thr Ala Asp Glu Lys Arg Val Ala
   405 410 415
   Ser Ile Ser Arg Leu Arg Glu Ala Phe Ser Leu His Pro Thr Lys Glu
   420 425 430
   Ile Lys Ser Arg Gly Pro Glu Thr Ala Glu Leu Thr Arg Ser Phe Pro
   435 440 445
   Ser Glu Lys Arg Gly Val Leu Ser Ser Tyr Pro Ser Asp Val Ile Ser
   450 455 460
   Tyr Arg Gly Leu Arg Gly Ser Gln Asp Lys Leu Val Ser Pro Thr Asp
   465 470 475 480
   Ser Pro Gly Asp Cys Met Asp Arg Glu Lys Ile Glu Lys Asp Ser Gly
   485 490 495
   Leu Ser Ser Thr Ser Ala Gly Ser Glu Glu Glu Phe Ser Thr Pro Glu
   500 505 510
   Val Ala Ser Ser Phe Ser Ser Asp Tyr Asn Val Ser Ser Leu Glu Asp
   515 520 525
   Arg Pro Ser Gln Glu Thr Ile Asn Cys Gly Asp Leu Asp Cys Arg Pro
   530 535 540
   Pro Gly Thr Gly Gln Ser Leu Lys Pro Glu Asp His Gly Tyr Gln Cys
   545 550 555 560
   Lys Ala Leu Pro Leu Ala Arg Leu Ser Pro Thr Asn Ala Lys Arg Phe
   565 570 575
   Lys Thr Glu Glu Arg Pro Ser Asn Val Asn Ile Ser Gln Arg Leu Pro
   580 585 590
   Gly Pro Gln Ser Thr Ser Ala Ala Glu Val Asp Val Ala Phe Lys Met
   595 600 605
   Asn Lys Arg Ile Val Leu Leu Glu Phe Ser Leu Ser Ser Leu Ala Lys
   610 615 620
   Arg Met Lys Gln Leu Gln His Leu Lys Ala Gln Asn Lys His Glu Leu
   625 630 635 640
   Ser Tyr Arg Lys Phe Arg Ala Lys Ile Cys Pro Gly Glu Asn Gln Ala
   645 650 655
   Ala Glu Asp Glu Leu Arg Lys Glu Ile Ser Lys Ser Met Phe Ala Glu
   660 665 670
   Met Glu Ile Leu Gly Gln Phe Asn Leu Gly Phe Ile Val Thr Lys Leu
   675 680 685
   Lys Glu Asp Leu Phe Leu Val Asp Gln His Ala Ala Asp Glu Lys Tyr
   690 695 700
   Asn Phe Glu Met Leu Gln Gln His Thr Val Leu Gln Ala Gln Arg Leu
   705 710 715 720
   Ile Thr Pro Gln Thr Leu Asn Leu Thr Ala Val Asn Glu Ala Val Leu
   725 730 735
   Ile Glu Asn Leu Glu Ile Phe Arg Lys Asn Gly Phe Asp Phe Val Ile
   740 745 750
   Asp Glu Asp Ala Pro Val Thr Glu Arg Ala Lys Leu Ile Ser Leu Pro
   755 760 765
   Thr Ser Lys Asn Trp Thr Phe Gly Pro Gln Asp Ile Asp Glu Leu Ile
   770 775 780
   Phe Met Leu Ser Asp Ser Pro Gly Val Met Cys Arg Pro Ser Arg Val
   785 790 795 800
   Arg Gln Met Phe Ala Ser Arg Ala Cys Arg Lys Ser Val Met Ile Gly
   805 810 815
   Thr Ala Leu Asn Ala Ser Glu Met Lys Lys Leu Ile Thr His Met Gly
   820 825 830
   Glu Met Asp His Pro Trp Asn Cys Pro His Gly Arg Pro Thr Met Arg
   835 840 845
   His Val Ala Asn Leu Asp Val Ile Ser Gln Asn
   850 855
<210> 6
   <211> 3056
   <212> DNA
   <213> Mus musculus
<400> 6
   gaattccggt gaaggtcctg aagaatttcc agattcctga gtatcattgg aggagacaga 60
   taacctgtcg tcaggtaacg atggtgtata tgcaacagaa atgggtgttc ctggagacgc 120
   gtcttttccc gagagcggca ccgcaactct cccgcggtga ctgtgactgg aggagtcctg 180
   catccatgga gcaaaccgaa ggcgtgagta cagaatgtgc taaggccatc aagcctattg 240
   atgggaagtc agtccatcaa atttgttctg ggcaggtgat actcagttta agcaccgctg 300
   tgaaggagtt gatagaaaat agtgtagatg ctggtgctac tactattgat ctaaggctta 360
   aagactatgg ggtggacctc attgaagttt cagacaatgg atgtggggta gaagaagaaa 420
   actttgaagg tctagctctg aaacatcaca catctaagat tcaagagttt gccgacctca 480
   cgcaggttga aactttcggc tttcgggggg aagctctgag ctctctgtgt gcactaagtg 540
   atgtcactat atctacctgc cacgggtctg caagcgttgg gactcgactg gtgtttgacc 600
   ataatgggaa aatcacccag aaaactccct acccccgacc taaaggaacc acagtcagtg 660
   tgcagcactt attttataca ctacccgtgc gttacaaaga gtttcagagg aacattaaaa 720
   aggagtattc caaaatggtg caggtcttac aggcgtactg tatcatctca gcaggcgtcc 780
   gtgtaagctg cactaatcag ctcggacagg ggaagcggca cgctgtggtg tgcacaagcg 840
   gcacgtctgg catgaaggaa aatatcgggt ctgtgtttgg ccagaagcag ttgcaaagcc 900
   tcattccttt tgttcagctg ccccctagtg acgctgtgtg tgaagagtac ggcctgagca 960
   cttcaggacg ccacaaaacc ttttctacgt ttcgggcttc atttcacagt gcacgcacgg 1020
   cgccgggagg agtgcaacag acaggcagtt tttcttcatc aatcagaggc cctgtgaccc 1080
   agcaaaggtc tctaagcttg tcaatgaggt tttatcacat gtataaccgg catcagtacc 1140
   catttgtcgt ccttaacgtt tccgttgact cagaatgtgt ggatattaat gtaactccag 1200
   ataaaaggca aattctacta caagaagaga agctattgct ggccgtttta aagacctcct 1260
   tgataggaat gtttgacagt gatgcaaaca agcttaatgt caaccagcag ccactgctag 1320
   atgttgaagg taacttagta aagctgcata ctgcagaact agaaaagcct gtgccaggaa 1380
   agcaagataa ctctccttca ctgaagagca cagcagacga gaaaagggta gcatccatct 1440
   ccaggctgag agaggccttt tctcttcatc ctactaaaga gatcaagtct aggggtccag
   1500
   agactgctga actgacacgg agttttccaa gtgagaaaag gggcgtgtta tcctcttatc
   1560
   cttcagacgt catctcttac agaggcctcc gtggctcgca ggacaaattg gtgagtccca
   1620
   cggacagccc tggtgactgt atggacagag agaaaataga aaaagactca gggctcagca
   1680
   gcacctcagc tggctctgag gaagagttca gcaccccaga agtggccagt agctttagca
   1740
   gtgactataa cgtgagctcc ctagaagaca gaccttctca ggaaaccata aactgtggtg
   1800
   acctggactg ccgtcctcca ggtacaggac agtccttgaa gccagaagac catggatatc
   1860
   aatgcaaagc tctacctcta gctcgtctgt cacccacaaa tgccaagcgc ttcaagacag
   1920
   aggaaagacc ctcaaatgtc aacatttctc aaagattgcc tggtcctcag agcacctcag
   1980
   cagctgaggt cgatgtagcc ataaaaatga ataagagaat cgtgctcctc gagttctctc
   2040
   tgagttctct agctaagcga atgaagcagt tacagcacct aaaggcgcag aacaaacatg
   2100
   aactgagtta cagaaaattt agggccaaga tttgccctgg agaaaaccaa gcagcagaag
   2160
   atgaactcag aaaagagatt agtaaatcga tgtttgcaga gatggagatc ttgggtcagt
   2220
   ttaacctggg atttatagta accaaactga aagaggacct cttcctggtg gaccagcatg
   2280
   ctgcggatga gaagtacaac tttgagatgc tgcagcagca cacggtgctc caggcgcaga
   2340
   ggctcatcac accccagact ctgaacttaa ctgctgtcaa tgaagctgta ctgatagaaa
   2400
   atctggaaat attcagaaag aatggctttg actttgtcat tgatgaggat gctccagtca
   2460
   ctgaaagggc taaattgatt tccttaccaa ctagtaaaaa ctggaccttt ggaccccaag
   2520
   atatagatga actgatcttt atgttaagtg acagccctgg ggtcatgtgc cggccctcac
   2580
   gagtcagaca gatgtttgct tccagagcct gtcggaagtc agtgatgatt ggaacggcgc
   2640
   tcaatgcgag cgagatgaag aagctcatca cccacatggg tgagatggac cacccctgga
   2700
   actgccccca cggcaggcca accatgaggc acgttgccaa tctggatgtc atctctcaga
   2760
   actgacacac cccttgtagc atagagttta ttacagattg ttcggtttgc aaagagaagg 2820
   ttttaagtaa tctgattatc gttgtacaaa aattagcatg ctgctttaat gtactggatc 2880
   catttaaaag cagtgttaag gcaggcatga tggagtgttc ctctagctca gctacttggg 2940
   tgatccggtg ggagctcatg tgagcccagg actttgagac cactccgagc cacattcatg 3000
   agactcaatt caaggacaaa aaaaaaaaga tatttttgaa gccttttaaa aaaaaa 3056
<210> 7
   <211> 862
   <212> PRT
   <213> Homo sapiens
<400> 7
   Met Glu Arg Ala Glu Ser Ser Ser Thr Glu Pro Ala Lys Ala Ile Lys
   1               5                   10                  15
   Pro Ile Asp Arg Lys Ser Val His Gln Ile Cys Ser Gly Gln Val Val
               20                  25                  30
   Leu Ser Leu Ser Thr Ala Val Lys Glu Leu Val Glu Asn Ser Leu Asp
           35                  40                  45
   Ala Gly Ala Thr Asn Ile Asp Leu Lys Leu Lys Asp Tyr Gly Val Asp
       50                  55                  60
   Leu Ile Glu Val Ser Asp Asn Gly Cys Gly Val Glu Glu Glu Asn Phe
   65                  70                  75                  80
   Glu Gly Leu Thr Leu Lys His His Thr Ser Lys Ile Gln Glu Phe Ala
                   85                  90                  95
   Asp Leu Thr Gln Val Glu Thr Phe Gly Phe Arg Gly Glu Ala Leu Ser
               100                 105                 110
   Ser Leu Cys Ala Leu Ser Asp Val Thr Ile Ser Thr Cys His Ala Ser
           115                 120                 125
   Ala Lys Val Gly Thr Arg Leu Met Phe Asp His Asn Gly Lys Ile Ile
       130                 135                 140
   Gln Lys Thr Pro Tyr Pro Arg Pro Arg Gly Thr Thr Val Ser Val Gln
   145                 150                 155                 160
   Gln Leu Phe Ser Thr Leu Pro Val Arg His Lys Glu Phe Gln Arg Asn
                   165                 170                 175
   Ile Lys Lys Glu Tyr Ala Lys Met Val Gln Val Leu His Ala Tyr Cys
               180                 185                 190
   Ile Ile Ser Ala Gly Ile Arg Val Ser Cys Thr Asn Gln Leu Gly Gln
           195                 200                 205
   Gly Lys Arg Gln Pro Val Val Cys Thr Gly Gly Ser Pro Ser Ile Lys
       210                 215                 220
   Glu Asn Ile Gly Ser Val Phe Gly Gln Lys Gln Leu Gln Ser Leu Ile
   225                 230                 235                 240
   Pro Phe Val Gln Leu Pro Pro Ser Asp Ser Val Cys Glu Glu Tyr Gly
                   245                 250                 255
   Leu Ser Cys Ser Asp Ala Leu His Asn Leu Phe Tyr Ile Ser Gly Phe
               260                 265                 270
   Ile Ser Gln Cys Thr His Gly Val Gly Arg Ser Ser Thr Asp Arg Gln
           275                 280                 285
   Phe Phe Phe Ile Asn Arg Arg Pro Cys Asp Pro Ala Lys Val Cys Arg
       290                 295                 300
   Leu Val Asn Glu Val Tyr His Met Tyr Asn Arg His Gln Tyr Pro Phe
   305                 310                 315                 320
   Val Val Leu Asn Ile Ser Val Asp Ser Glu Cys Val Asp Ile Asn Val
                   325                 330                 335
   Thr Pro Asp Lys Arg Gln Ile Leu Leu Gln Glu Glu Lys Leu Leu Leu
               340                 345                 350
   Ala Val Leu Lys Thr Ser Leu Ile Gly Met Phe Asp Ser Asp Val Asn
           355                 360                 365
   Lys Leu Asn Val Ser Gln Gln Pro Leu Leu Asp Val Glu Gly Asn Leu
       370                 375                 380
   Ile Lys Met His Ala Ala Asp Leu Glu Lys Pro Met Val Glu Lys Gln
   385                 390                 395                 400
   Asp Gln Ser Pro Ser Leu Arg Thr Gly Glu Glu Lys Lys Asp Val Ser
                   405                 410                 415
   Ile Ser Arg Leu Arg Glu Ala Phe Ser Leu Arg His Thr Thr Glu Asn
               420                 425                 430
   Lys Pro His Ser Pro Lys Thr Pro Glu Pro Arg Arg Ser Pro Leu Gly
           435                 440                 445
   Gln Lys Arg Gly Met Leu Ser Ser Ser Thr Ser Gly Ala Ile Ser Asp
       450                 455                 460
   Lys Gly Val Leu Arg Pro Gln Lys Glu Ala Val Ser Ser Ser His Gly
   465                 470                 475                 480
   Pro Ser Asp Pro Thr Asp Arg Ala Glu Val Glu Lys Asp Ser Gly His
                   485                 490                 495
   Gly Ser Thr Ser Val Asp Ser Glu Gly Phe Ser Ile Pro Asp Thr Gly
               500                 505                 510
   Ser His Cys Ser Ser Glu Tyr Ala Ala Ser Ser Pro Gly Asp Arg Gly
           515                 520                 525
   Ser Gln Glu His Val Asp Ser Gln Glu Lys Ala Pro Glu Thr Asp Asp
       530                 535                 540
   Ser Phe Ser Asp Val Asp Cys His Ser Asn Gln Glu Asp Thr Gly Cys
   545                 550                 555                 560
   Lys Phe Arg Val Leu Pro Gln Pro Thr Asn Leu Ala Thr Pro Asn Thr
                   565                 570                 575
   Lys Arg Phe Lys Lys Glu Glu Ile Leu Ser Ser Ser Asp Ile Cys Gln
               580                 585                 590
   Lys Leu Val Asn Thr Gln Asp Met Ser Ala Ser Gln Val Asp Val Ala
           595                 600                 605
   Val Lys Ile Asn Lys Lys Val Val Pro Leu Asp Phe Ser Met Ser Ser
       610                 615                 620
   Leu Ala Lys Arg Ile Lys Gln Leu His His Glu Ala Gln Gln Ser Glu
   625                 630                 635                 640
   Gly Glu Gln Asn Tyr Arg Lys Phe Arg Ala Lys Ile Cys Pro Gly Glu
                   645                 650                 655
   Asn Gln Ala Ala Glu Asp Glu Leu Arg Lys Glu Ile Ser Lys Thr Met
               660                 665                 670
   Phe Ala Glu Met Glu Ile Ile Gly Gln Phe Asn Leu Gly Phe Ile Ile
           675                 680                 685
   Thr Lys Leu Asn Glu Asp Ile Phe Ile Val Asp Gln His Ala Thr Asp
       690                 695                 700
   Glu Lys Tyr Asn Phe Glu Met Leu Gln Gln His Thr Val Leu Gln Gly
   705                 710                 715                 720
   Gln Arg Leu Ile Ala Pro Gln Thr Leu Asn Leu Thr Ala Val Asn Glu
                   725                 730                 735
   Ala Val Leu Ile Glu Asn Leu Glu Ile Phe Arg Lys Asn Gly Phe Asp
               740                 745                 750
   Phe Val Ile Asp Glu Asn Ala Pro Val Thr Glu Arg Ala Lys Leu Ile
           755                 760                 765
   Ser Leu Pro Thr Ser Lys Asn Trp Thr Phe Gly Pro Gln Asp Val Asp
       770                 775                 780
   Glu Leu Ile Phe Met Leu Ser Asp Ser Pro Gly Val Met Cys Arg Pro
   785                 790                 795                 800
   Ser Arg Val Lys Gln Met Phe Ala Ser Arg Ala Cys Arg Lys Ser Val
                   805                 810                 815
   Met Ile Gly Thr Ala Leu Asn Thr Ser Glu Met Lys Lys Leu Ile Thr
               820                 825                 830
   His Met Gly Glu Met Asp His Pro Trp Asn Cys Pro His Gly Arg Pro
           835                 840                 845
   Thr Met Arg His Ile Ala Asn Leu Gly Val Ile Ser Gln Asn
       850                 855                 860
<210> 8
   <211> 2771
   <212> DNA
   <213> Homo sapiens
<400> 8
   cgaggcggat cgggtgttgc atccatggag cgagctgaga gctcgagtac agaacctgct
   60
   aaggccatca aacctattga tcggaagtca gtccatcaga tttgctctgg gcaggtggta
   120
   ctgagtctaa gcactgcggt aaaggagtta gtagaaaaca gtctggatgc tggtgccact
   180
   aatattgatc taaagcttaa ggactatgga gtggatctta ttgaagtttc agacaatgga
   240
   tgtggggtag aagaagaaaa cttcgaaggc ttaactctga aacatcacac atctaagatt
   300
   caagagtttg ccgacctaac tcaggttgaa acttttggct ttcgggggga agctctgagc 360
   tcactttgtg cactgagcga tgtcaccatt tctacctgcc acgcatcggc gaaggttgga 420
   actcgactga tgtttgatca caatgggaaa attatccaga aaacccccta cccccgcccc 480
   agagggacca cagtcagcgt gcagcagtta ttttccacac tacctgtgcg ccataaggaa 540
   tttcaaagga atattaagaa ggagtatgcc aaaatggtcc aggtcttaca tgcatactgt 600
   atcatttcag caggcatccg tgtaagttgc accaatcagc ttggacaagg aaaacgacag 660
   cctgtggtat gcacaggtgg aagccccagc ataaaggaaa atatcggctc tgtgtttggg 720
   cagaagcagt tgcaaagcct cattcctttt gttcagctgc cccctagtga ctccgtgtgt 780
   gaagagtacg gtttgagctg ttcggatgct ctgcataatc ttttttacat ctcaggtttc 840
   atttcacaat gcacgcatgg agttggaagg agttcaacag acagacagtt tttctttatc 900
   aaccggcggc cttgtgaccc agcaaaggtc tgcagactcg tgaatgaggt ctaccacatg 960
   tataatcgac accagtatcc atttgttgtt cttaacattt ctgttgattc agaatgcgtt 1020
   gatatcaatg ttactccaga taaaaggcaa attttgctac aagaggaaaa gcttttgttg 1080
   gcagttttaa agacctcttt gataggaatg tttgatagtg atgtcaacaa gctaaatgtc 1140
   agtcagcagc cactgctgga tgttgaaggt aacttaataa aaatgcatgc agcggatttg 1200
   gaaaagccca tggtagaaaa gcaggatcaa tccccttcat taaggactgg agaagaaaaa 1260
   aaagacgtgt ccatttccag actgcgagag gccttttctc ttcgtcacac aacagagaac 1320
   aagcctcaca gcccaaagac tccagaacca agaaggagcc ctctaggaca gaaaaggggt 1380
   atgctgtctt ctagcacttc aggtgccatc tctgacaaag gcgtcctgag acctcagaaa 1440
   gaggcagtga gttccagtca cggacccagt gaccctacgg acagagcgga ggtggagaag 1500
   gactcggggc acggcagcac ttccgtggat tctgaggggt tcagcatccc agacacgggc 1560
   agtcactgca gcagcgagta tgcggccagc tccccagggg acaggggctc gcaggaacat 1620
   gtggactctc aggagaaagc gcctgaaact gacgactctt tttcagatgt ggactgccat
   1680
   tcaaaccagg aagataccgg atgtaaattt cgagttttgc ctcagccaac taatctcgca
   1740
   accccaaaca caaagcgttt taaaaaagaa gaaattcttt ccagttctga catttgtcaa
   1800
   aagttagtaa atactcagga catgtcagcc tctcaggttg atgtagctgt gaaaattaat
   1860
   aagaaagttg tgcccctgga cttttctatg agttctttag ctaaacgaat aaagcagtta
   1920
   catcatgaag cacagcaaag tgaaggggaa cagaattaca ggaagtttag ggcaaagatt
   1980
   tgtcctggag aaaatcaagc agccgaagat gaactaagaa aagagataag taaaacgatg
   2040
   tttgcagaaa tggaaatcat tggtcagttt aacctgggat ttataataac caaactgaat
   2100
   gaggatatct tcatagtgga ccagcatgcc acggacgaga agtataactt cgagatgctg
   2160
   cagcagcaca ccgtgctcca ggggcagagg ctcatagcac ctcagactct caacttaact
   2220
   gctgttaatg aagctgttct gatagaaaat ctggaaatat ttagaaagaa tggctttgat
   2280
   tttgttatcg atgaaaatgc tccagtcact gaaagggcta aactgatttc cttgccaact
   2340
   agtaaaaact ggaccttcgg accccaggac gtcgatgaac tgatcttcat gctgagcgac
   2400
   agccctgggg tcatgtgccg gccttcccga gtcaagcaga tgtttgcctc cagagcctgc
   2460
   cggaagtcgg tgatgattgg gactgctctt aacacaagcg agatgaagaa actgatcacc
   2520
   cacatggggg agatggacca cccctggaac tgtccccatg gaaggccaac catgagacac
   2580
   atcgccaacc tgggtgtcat ttctcagaac tgaccgtagt cactgtatgg aataattggt
   2640
   tttatcgcag atttttatgt tttgaaagac agagtcttca ctaacctttt ttgttttaaa
   2700
   atgaaacctg ctacttaaaa aaaatacaca tcacacccat ttaaaagtga tcttgagaac
   2760
   cttttcaaac c
   2771
<210> 9
   <211> 932
   <212> PRT
   <213> Homo sapiens
<400> 9
   Met Lys Gln Leu Pro Ala Ala Thr Val Arg Leu Leu Ser Ser Ser Gln
   1 5 10 15
   Ile Ile Thr Ser Val Val Ser Val Val Lys Glu Leu Ile Glu Asn Ser
   20 25 30
   Leu Asp Ala Gly Ala Thr Ser Val Asp Val Lys Leu Glu Asn Tyr Gly
   35 40 45
   Phe Asp Lys Ile Glu Val Arg Asp Asn Gly Glu Gly Ile Lys Ala Val
   50 55 60
   Asp Ala Pro Val Met Ala Met Lys Tyr Tyr Thr Ser Lys Ile Asn Ser
   65 70 75 80
   His Glu Asp Leu Glu Asn Leu Thr Thr Tyr Gly Phe Arg Gly Glu Ala
   85 90 95
   Leu Gly Ser Ile Cys Cys Ile Ala Glu Val Leu Ile Thr Thr Arg Thr
   100 105 110
   Ala Ala Asp Asn Phe Ser Thr Gln Tyr Val Leu Asp Gly Ser Gly His
   115 120 125
   Ile Leu Ser Gln Lys Pro Ser His Leu Gly Gln Gly Thr Thr Val Thr
   130 135 140
   Ala Leu Arg Leu Phe Lys Asn Leu Pro Val Arg Lys Gln Phe Tyr Ser
   145 150 155 160
   Thr Ala Lys Lys Cys Lys Asp Glu Ile Lys Lys Ile Gln Asp Leu Leu
   165 170 175
   Met Ser Phe Gly Ile Leu Lys Pro Asp Leu Arg Ile Val Phe Val His
   180 185 190
   Asn Lys Ala Val Ile Trp Gln Lys Ser Arg Val Ser Asp His Lys Met
   195 200 205
   Ala Leu Met Ser Val Leu Gly Thr Ala Val Met Asn Asn Met Glu Ser
   210 215 220
   Phe Gln Tyr His Ser Glu Glu Ser Gln Ile Tyr Leu Ser Gly Phe Leu
   225 230 235 240
   Pro Lys Cys Asp Ala Asp His Ser Phe Thr Ser Leu Ser Thr Pro Glu
   245 250 255
   Arg Ser Phe Ile Phe Ile Asn Ser Arg Pro Val His Gln Lys Asp Ile
   260 265 270
   Leu Lys Leu Ile Arg His His Tyr Asn Leu Lys Cys Leu Lys Glu Ser
   275 280 285
   Thr Arg Leu Tyr Pro Val Phe Phe Leu Lys Ile Asp Val Pro Thr Ala
   290 295 300
   Asp Val Asp Val Asn Leu Thr Pro Asp Lys Ser Gln Val Leu Leu Gln
   305 310 315 320
   Asn Lys Glu Ser Val Leu Ile Ala Leu Glu Asn Leu Met Thr Thr Cys
   325 330 335
   Tyr Gly Pro Leu Pro Ser Thr Asn Ser Tyr Glu Asn Asn Lys Thr Asp
   340 345 350
   Val Ser Ala Ala Asp Phe Val Leu Ser Lys Thr Ala Glu Thr Asp Val
   355 360 365
   Leu Phe Asn Lys Val Glu Ser Ser Gly Lys Asn Tyr Ser Asn Val Asp
   370 375 380
   Thr Ser Val Phe Pro Phe Gln Asn Asp Met His Asn Asp Glu Ser Gly
   385 390 395 400
   Lys Asn Thr Asp Asp Cys Leu Asn His Gln Phe Ser Phe Gly Asp Phe
   405 410 415
   Gly Tyr Gly His Cys Ser Ser Glu Phe Ser Asn Phe Asp Lys Asn Thr
   420 425 430
   Lys Asn Ala Phe Gln Asp Phe Ser Met Ser Asn Val Ser Trp Glu Asn
   435 440 445
   Ser Gln Thr Glu Tyr Ser Lys Thr Cys Phe Phe Ser Ser Val Lys His
   450 455 460
   Thr Gln Ser Glu Asn Gly Asn Lys Asp His Phe Asp Glu Ser Gly Glu
   465 470 475 480
   Asn Glu Glu Glu Ala Gly Leu Glu Asn Ser Ser Glu Phe Ser Ala Asp
   485 490 495
   Glu Trp Ser Arg Gly Asn Phe Leu Lys Asn Ser Val Gly Glu Asn Phe
   500 505 510
   Glu Pro Val Lys Ile Leu Val Pro Glu Lys Ser Leu Pro Cys Lys Val
   515 520 525
   Ser Asn Asn Asn Tyr Pro Ile Pro Glu Gln Met Asn Leu Asn Glu Asp
   530 535 540
   Ser Cys Asn Lys Lys Ser Asn Val Ile Asp Asn Lys Ser Gly Lys Val
   545 550 555 560
   Thr Ala Tyr Asp Leu Leu Ser Asn Arg Val Ile Lys Lys Pro Met Ser
   565 570 575
   Ala Ser Ala Leu Phe Val Gln Asp His Arg Pro Gln Phe Leu Ile Glu
   580 585 590
   Asn Pro Lys Thr Ser Leu Glu Asp Ala Thr Leu Gln Ile Glu Glu Leu
   595 600 605
   Trp Lys Thr Leu Ser Glu Glu Glu Lys Leu Lys Tyr Glu Glu Lys Ala
   610 615 620
   Thr Lys Asp Leu Glu Arg Tyr Asn Ser Gln Met Lys Arg Ala Ile Glu
   625 630 635 640
   Gln Glu Ser Gln Met Ser Leu Lys Asp Gly Arg Lys Lys Ile Lys Pro
   645 650 655
   Thr Ser Ala Trp Asn Leu Ala Gln Lys His Lys Leu Lys Thr Ser Leu
   660 665 670
   Ser Asn Gln Pro Lys Leu Asp Glu Leu Leu Gln Ser Gln Ile Glu Lys
   675 680 685
   Arg Arg Ser Gln Asn Ile Lys Met Val Gln Ile Pro Phe Ser Met Lys
   690 695 700
   Asn Leu Lys Ile Asn Phe Lys Lys Gln Asn Lys Val Asp Leu Glu Glu
   705 710 715 720
   Lys Asp Glu Pro Cys Leu Ile His Asn Leu Arg Phe Pro Asp Ala Trp
   725 730 735
   Leu Met Thr Ser Lys Thr Glu Val Met Leu Leu Asn Pro Tyr Arg Val
   740 745 750
   Glu Glu Ala Leu Leu Phe Lys Arg Leu Leu Glu Asn His Lys Leu Pro
   755 760 765
   Ala Glu Pro Leu Glu Lys Pro Ile Met Leu Thr Glu Ser Leu Phe Asn
       770                 775                 780
   Gly Ser His Tyr Leu Asp Val Leu Tyr Lys Met Thr Ala Asp Asp Gln
   785                 790                 795                 800
   Arg Tyr Ser Gly Ser Thr Tyr Leu Ser Asp Pro Arg Leu Thr Ala Asn
                   805                 810                 815
   Gly Phe Lys Ile Lys Leu Ile Pro Gly Val Ser Ile Thr Glu Asn Tyr
               820                 825                 830
   Leu Glu Ile Glu Gly Met Ala Asn Cys Leu Pro Phe Tyr Gly Val Ala
           835                 840                 845
   Asp Leu Lys Glu Ile Leu Asn Ala Ile Leu Asn Arg Asn Ala Lys Glu
       850                 855                 860
   Val Tyr Glu Cys Arg Pro Arg Lys Val Ile Ser Tyr Leu Glu Gly Glu
   865                 870                 875                 880
   Ala Val Arg Leu Ser Arg Gln Leu Pro Met Tyr Leu Ser Lys Glu Asp
                   885                 890                 895
   Ile Gln Asp Ile Ile Tyr Arg Met Lys His Gln Phe Gly Asn Glu Ile
               900                 905                 910
   Lys Glu Cys Val His Gly Arg Pro Phe Phe His His Leu Thr Tyr Leu
           915                 920                 925
   Pro Glu Thr Thr
       930
<210> 10
   <211> 3063
   <212> DNA
   <213> Homo sapiens
<400> 10
   ggcacgagtg gctgcttgcg gctagtggat ggtaattgcc tgcctcgcgc tagcagcaag 60
   ctgctctgtt aaaagcgaaa atgaaacaat tgcctgcggc aacagttcga ctcctttcaa 120
   gttctcagat catcacttcg gtggtcagtg ttgtaaaaga gcttattgaa aactccttgg 180
   atgctggtgc cacaagcgta gatgttaaac tggagaacta tggatttgat aaaattgagg 240
   tgcgagataa cggggagggt atcaaggctg ttgatgcacc tgtaatggca atgaagtact 300
   acacctcaaa aataaatagt catgaagatc ttgaaaattt gacaacttac ggttttcgtg 360
   gagaagcctt ggggtcaatt tgttgtatag ctgaggtttt aattacaaca agaacggctg 420
   ctgataattt tagcacccag tatgttttag atggcagtgg ccacatactt tctcagaaac 480
   cttcacatct tggtcaaggt acaactgtaa ctgctttaag attatttaag aatctacctg 540
   taagaaagca gttttactca actgcaaaaa aatgtaaaga tgaaataaaa aagatccaag 600
   atctcctcat gagctttggt atccttaaac ctgacttaag gattgtcttt gtacataaca 660
   aggcagttat ttggcagaaa agcagagtat cagatcacaa gatggctctc atgtcagttc 720
   tggggactgc tgttatgaac aatatggaat cctttcagta ccactctgaa gaatctcaga 780
   tttatctcag tggatttctt ccaaagtgtg atgcagacca ctctttcact agtctttcaa 840
   caccagaaag aagtttcatc ttcataaaca gtcgaccagt acatcaaaaa gatatcttaa 900
   agttaatccg acatcattac aatctgaaat gcctaaagga atctactcgt ttgtatcctg 960
   ttttctttct gaaaatcgat gttcctacag ctgatgttga tgtaaattta acaccagata 1020
   aaagccaagt attattacaa aataaggaat ctgttttaat tgctcttgaa aatctgatga 1080
   cgacttgtta tggaccatta cctagtacaa attcttatga aaataataaa acagatgttt 1140
   ccgcagctga catcgttctt agtaaaacag cagaaacaga tgtgcttttt aataaagtgg 1200
   aatcatctgg aaagaattat tcaaatgttg atacttcagt cattccattc caaaatgata 1260
   tgcataatga tgaatctgga aaaaacactg atgattgttt aaatcaccag ataagtattg 1320
   gtgactttgg ttatggtcat tgtagtagtg aaatttctaa cattgataaa aacactaaga 1380
   atgcatttca ggacatttca atgagtaatg tatcatggga gaactctcag acggaatata 1440
   gtaaaacttg ttttataagt tccgttaagc acacccagtc agaaaatggc aataaagacc 1500
   atatagatga gagtggggaa aatgaggaag aagcaggtct tgaaaactct tcggaaattt 1560
   ctgcagatga gtggagcagg ggaaatatac ttaaaaattc agtgggagag aatattgaac
   1620
   ctgtgaaaat tttagtgcct gaaaaaagtt taccatgtaa agtaagtaat aataattatc
   1680
   caatccctga acaaatgaat cttaatgaag attcatgtaa caaaaaatca aatgtaatag
   1740
   ataataaatc tggaaaagtt acagcttatg atttacttag caatcgagta atcaagaaac
   1800
   ccatgtcagc aagtgctctt tttgttcaag atcatcgtcc tcagtttctc atagaaaatc
   1860
   ctaagactag tttagaggat gcaacactac aaattgaaga actgtggaag acattgagtg
   1920
   aagaggaaaa actgaaatat gaagagaagg ctactaaaga cttggaacga tacaatagtc
   1980
   aaatgaagag agccattgaa caggagtcac aaatgtcact aaaagatggc agaaaaaaga
   2040
   taaaacccac cagcgcatgg aatttggccc agaagcacaa gttaaaaacc tcattatcta
   2100
   atcaaccaaa acttgatgaa ctccttcagt cccaaattga aaaaagaagg agtcaaaata
   2160
   ttaaaatggt acagatcccc ttttctatga aaaacttaaa aataaatttt aagaaacaaa
   2220
   acaaagttga cttagaagag aaggatgaac cttgcttgat ccacaatctc aggtttcctg
   2280
   atgcatggct aatgacatcc aaaacagagg taatgttatt aaatccatat agagtagaag
   2340
   aagccctgct atttaaaaga cttcttgaga atcataaact tcctgcagag ccactggaaa
   2400
   agccaattat gttaacagag agtcttttta atggatctca ttatttagac gttttatata
   2460
   aaatgacagc agatgaccaa agatacagtg gatcaactta cctgtctgat cctcgtctta
   2520
   cagcgaatgg tttcaagata aaattgatac caggagtttc aattactgaa aattacttgg
   2580
   aaatagaagg aatggctaat tgtctcccat tctatggagt agcagattta aaagaaattc
   2640
   ttaatgctat attaaacaga aatgcaaagg aagtttatga atgtagacct cgcaaagtga
   2700
   taagttattt agagggagaa gcagtgcgtc tatccagaca attacccatg tacttatcaa
   2760
   aagaggacat ccaagacatt atctacagaa tgaagcacca gtttggaaat gaaattaaag
   2820
   agtgtgttca tggtcgccca ttttttcatc atttaaccta tcttccagaa actacatgat
   2880
   taaatatgtt taagaagatt agttaccatt gaaattggtt ctgtcataaa acagcatgag
   2940
   tctggtttta aattatcttt gtattatgtg tcacatggtt attttttaaa tgaggattca
   3000
   ctgacttgtt tttatattga aaaaagttcc acgtattgta gaaaacgtaa ataaactaat
   3060
   aac
   3063
<210> 11
   <211> 934
   <212> PRT
   <213> Homo sapiens
<400> 11
   Met Ala Val Gln Pro Lys Glu Thr Leu Gln Leu Glu Ser Ala Ala Glu
   1 5 10 15
   Val Gly Phe Val Arg Phe Phe Gln Gly Met Pro Glu Lys Pro Thr Thr
   20 25 30
   Thr Val Arg Leu Phe Asp Arg Gly Asp Phe Tyr Thr Ala His Gly Glu
   35 40 45
   Asp Ala Leu Leu Ala Ala Arg Glu Val Phe Lys Thr Gln Gly Val Ile
   50 55 60
   Lys Tyr Met Gly Pro Ala Gly Ala Lys Asn Leu Gln Ser Val Val Leu
   65 70 75 80
   Ser Lys Met Asn Phe Glu Ser Phe Val Lys Asp Leu Leu Leu Val Arg
   85 90 95
   Gln Tyr Arg Val Glu Val Tyr Lys Asn Arg Ala Gly Asn Lys Ala Ser
   100 105 110
   Lys Glu Asn Asp Trp Tyr Leu Ala Tyr Lys Ala Ser Pro Gly Asn Leu
   115 120 125
   Ser Gln Phe Glu Asp Ile Leu Phe Gly Asn Asn Asp Met Ser Ala Ser
   130 135 140
   Phe Gly Val Val Gly Val Lys Met Ser Ala Val Asp Gly Gln Arg Gln
   145 150 155 160
   Val Gly Val Gly Tyr Val Asp Ser Phe Gln Arg Lys Leu Gly Leu Cys
   165 170 175
   Glu Phe Pro Asp Asn Asp Gln Phe Ser Asn Leu Glu Ala Leu Leu Phe
   180 185 190
   Gln Phe Gly Pro Lys Glu Cys Val Leu Pro Gly Gly Glu Thr Ala Gly
   195 200 205
   Asp Met Gly Lys Leu Arg Gln Phe Phe Gln Arg Gly Gly Phe Leu Phe
   210 215 220
   Thr Glu Arg Lys Lys Ala Asp Phe Ser Thr Lys Asp Phe Tyr Gln Asp
   225 230 235 240
   Leu Asn Arg Leu Leu Lys Gly Lys Lys Gly Glu Gln Met Asn Ser Ala
   245 250 255
   Val Leu Pro Glu Met Glu Asn Gln Val Ala Val Ser Ser Leu Ser Ala
   260 265 270
   Val Ile Lys Phe Leu Glu Leu Leu Ser Asp Asp Ser Asn Phe Gly Gln
   275 280 285
   Phe Glu Leu Thr Thr Phe Asp Phe Ser Gln Tyr Met Lys Leu Asp Ile
   290 295 300
   Ala Ala Val Arg Ala Leu Asn Leu Phe Gln Gly Ser Val Glu Asp Thr
   305 310 315 320
   Thr Gly Ser Gln Ser Leu Ala Ala Leu Leu Asn Lys Cys Lys Thr Pro
   325 330 335
   Gln Gly Gln Arg Leu Val Asn Gln Trp Ile Lys Gln Pro Leu Met Asp
   340 345 350
   Lys Asn Arg Ile Glu Glu Arg Leu Asn Leu Val Glu Ala Phe Val Glu
   355 360 365
   Asp Ala Glu Leu Arg Gln Thr Leu Gln Glu Asp Leu Leu Arg Arg Phe
   370 375 380
   Pro Asp Leu Asn Arg Leu Ala Lys Lys Phe Gln Arg Gln Ala Ala Asn
   385 390 395 400
   Leu Gln Asp Cys Tyr Arg Leu Tyr Gln Gly Ile Asn Gln Leu Pro Asn
   405 410 415
   Val Ile Gln Ala Leu Glu Lys His Glu Gly Lys His Gln Lys Leu Leu
   420 425 430
   Leu Ala Val Phe Val Thr Pro Leu Thr Asp Leu Arg Ser Asp Phe Ser
   435 440 445
   Lys Phe Gln Glu Met Ile Glu Thr Thr Leu Asp Met Asp Gln Val Glu
   450 455 460
   Asn His Glu Phe Leu Val Lys Pro Ser Phe Asp Pro Asn Leu Ser Glu
   465 470 475 480
   Leu Arg Glu Ile Met Asn Asp Leu Glu Lys Lys Met Gln Ser Thr Leu
   485 490 495
   Ile Ser Ala Ala Arg Asp Leu Gly Leu Asp Pro Gly Lys Gln Ile Lys
   500 505 510
   Leu Asp Ser Ser Ala Gln Phe Gly Tyr Tyr Phe Arg Val Thr Cys Lys
   515 520 525
   Glu Glu Lys Val Leu Arg Asn Asn Lys Asn Phe Ser Thr Val Asp Ile
   530 535 540
   Gln Lys Asn Gly Val Lys Phe Thr Asn Ser Lys Leu Thr Ser Leu Asn
   545 550 555 560
   Glu Glu Tyr Thr Lys Asn Lys Thr Glu Tyr Glu Glu Ala Gln Asp Ala
   565 570 575
   Ile Val Lys Glu Ile Val Asn Ile Ser Ser Gly Tyr Val Glu Pro Met
   580 585 590
   Gln Thr Leu Asn Asp Val Leu Ala Gln Leu Asp Ala Val Val Ser Phe
   595 600 605
   Ala His Val Ser Asn Gly Ala Pro Val Pro Tyr Val Arg Pro Ala Ile
   610 615 620
   Leu Glu Lys Gly Gln Gly Arg Ile Ile Leu Lys Ala Ser Arg His Ala
   625 630 635 640
   Cys Val Glu Val Gln Asp Glu Ile Ala Phe Ile Pro Asn Asp Val Tyr
   645 650 655
   Phe Glu Lys Asp Lys Gln Met Phe His Ile Ile Thr Gly Pro Asn Met
   660 665 670
   Gly Gly Lys Ser Thr Tyr Phe Arg Gln Thr Gly Val Phe Val Leu Met
   675 680 685
   Ala Gln Phe Gly Cys Phe Val Pro Cys Glu Ser Ala Glu Val Ser Phe
   690 695 700
   Val Asp Cys Phe Leu Ala Arg Val Gly Ala Gly Asp Ser Gln Leu Lys
   705 710 715 720
   Gly Val Ser Thr Phe Met Ala Glu Met Leu Glu Thr Ala Ser Phe Leu
   725 730 735
   Arg Ser Ala Thr Lys Asp Ser Leu Phe Phe Phe Asp Glu Leu Gly Arg
   740 745 750
   Gly Thr Ser Thr Tyr Asp Gly Phe Gly Leu Ala Trp Ala Ile Ser Glu
   755 760 765
   Tyr Ile Ala Thr Lys Ile Gly Ala Phe Cys Met Phe Ala Thr His Phe
   770 775 780
   His Glu Leu Thr Ala Leu Ala Asn Gln Ile Pro Thr Val Asn Asn Leu
   785 790 795 800
   His Val Thr Ala Leu Thr Thr Glu Glu Thr Leu Thr Met Leu Tyr Gln
   805 810 815
   Val Lys Lys Gly Val Cys Asp Gln Ser Phe Gly Phe His Val Ala Glu
   820 825 830
   Leu Ala Asn Phe Pro Lys His Val Phe Glu Cys Ala Lys Gln Lys Ala
   835 840 845
   Leu Glu Leu Glu Glu Phe Gln Tyr Phe Gly Glu Ser Gln Gly Tyr Asp
   850 855 860
   Phe Met Glu Pro Ala Ala Lys Lys Cys Tyr Leu Glu Arg Glu Gln Gly
   865 870 875 880
   Glu Lys Phe Phe Gln Glu Phe Leu Ser Lys Val Lys Gln Met Pro Phe
   885 890 895
   Thr Glu Met Ser Glu Glu Asn Phe Thr Phe Lys Leu Lys Gln Leu Lys
   900 905 910
   Ala Glu Val Phe Ala Lys Asn Asn Ser Phe Val Asn Glu Phe Phe Ser
   915 920 925
   Arg Phe Lys Val Thr Thr
   930
<210> 12
   <211> 3145
   <212> DNA
   <213> Homo sapiens
<400> 12
   ggcgggaaac agcttagtgg gtgtggggtc gcgcattttc ttcaaccagg aggtgaggag 60
   gtttcgacat ggcggtgcag ccgaaggaga cgctgcagtt ggagagcgcg gccgaggtcg 120
   gcttcgtgcg cttctttcag ggcatgccgg agaagccgac caccacagtg cgccttttcg 180
   accggggcga cttctatacg gcgcacggcg aggacgcgct gctggccgcc cgggaggtgt 240
   tcaagaccca gggggtgatc aagtacatgg ggccggcagg agcaaagaat ctgcagagtg 300
   ttgtgcttag taaaatgaat tttgaatctt ttgtaaaaga tcttcttctg gttcgtcagt 360
   atagagttga agtttataag aatagagctg gaaataaggc atccaaggag aatgattggt 420
   atttggcata taaggcttct cctggcaatc tctctcagtt tgaagacatt ctctttggta 480
   acaatgatat gtcagcttcc attggtgttg tgggtgttaa aatgtccgca gttgatggcc 540
   agagacaggt tggagttggg tatgtggatt ccatacagag gaaactagga ctgtgtgaat 600
   tccctgataa tgatcagttc tccaatcttg aggctctcct catccagatt ggaccaaagg 660
   aatgtgtttt acccggagga gagactgctg gagacatggg gaaactgaga cagataattc 720
   aaagaggagg aattctgatc acagaaagaa aaaaagctga cttttccaca aaagacattt 780
   atcaggacct caaccggttg ttgaaaggca aaaagggaga gcagatgaat agtgctgtat 840
   tgccagaaat ggagaatcag gttgcagttt catcactgtc tgcggtaatc aagtttttag 900
   aactcttatc agatgattcc aactttggac agtttgaact gactactttt gacttcagcc 960
   agtatatgaa attggatatt gcagcagtca gagcccttaa cctttttcag ggttctgttg 1020
   aagataccac tggctctcag tctctggctg ccttgctgaa taagtgtaaa acccctcaag 1080
   gacaaagact tgttaaccag tggattaagc agcctctcat ggataagaac agaatagagg 1140
   agagattgaa tttagtggaa gcttttgtag aagatgcaga attgaggcag actttacaag 1200
   aagatttact tcgtcgattc ccagatctta accgacttgc caagaagttt caaagacaag 1260
   cagcaaactt acaagattgt taccgactct atcagggtat aaatcaacta cctaatgtta 1320
   tacaggctct ggaaaaacat gaaggaaaac accagaaatt attgttggca gtttttgtga 1380
   ctcctcttac tgatcttcgt tctgacttct ccaagtttca ggaaatgata gaaacaactt 1440
   tagatatgga tcaggtggaa aaccatgaat tccttgtaaa accttcattt gatcctaatc 1500
   tcagtgaatt aagagaaata atgaatgact tggaaaagaa gatgcagtca acattaataa 1560
   gtgcagccag agatcttggc ttggaccctg gcaaacagat taaactggat tccagtgcac 1620
   agtttggata ttactttcgt gtaacctgta aggaagaaaa agtccttcgt aacaataaaa 1680
   actttagtac tgtagatatc cagaagaatg gtgttaaatt taccaacagc aaattgactt 1740
   ctttaaatga agagtatacc aaaaataaaa cagaatatga agaagcccag gatgccattg 1800
   ttaaagaaat tgtcaatatt tcttcaggct atgtagaacc aatgcagaca ctcaatgatg 1860
   tgttagctca gctagatgct gttgtcagct ttgctcacgt gtcaaatgga gcacctgttc 1920
   catatgtacg accagccatt ttggagaaag gacaaggaag aattatatta aaagcatcca 1980
   ggcatgcttg tgttgaagtt caagatgaaa ttgcatttat tcctaatgac gtatactttg 2040
   aaaaagataa acagatgttc cacatcatta ctggccccaa tatgggaggt aaatcaacat 2100
   atattcgaca aactggggtg atagtactca tggcccaaat tgggtgtttt gtgccatgtg 2160
   agtcagcaga agtgtccatt gtggactgca tcttagcccg agtaggggct ggtgacagtc 2220
   aattgaaagg agtctccacg ttcatggctg aaatgttgga aactgcttct atcctcaggt 2280
   ctgcaaccaa agattcatta ataatcatag atgaattggg aagaggaact tctacctacg 2340
   atggatttgg gttagcatgg gctatatcag aatacattgc aacaaagatt ggtgcttttt 2400
   gcatgtttgc aacccatttt catgaactta ctgccttggc caatcagata ccaactgtta 2460
   ataatctaca tgtcacagca ctcaccactg aagagacctt aactatgctt tatcaggtga 2520
   agaaaggtgt ctgtgatcaa agttttggga ttcatgttgc agagcttgct aatttcccta 2580
   agcatgtaat agagtgtgct aaacagaaag ccctggaact tgaggagttt cagtatattg 2640
   gagaatcgca aggatatgat atcatggaac cagcagcaaa gaagtgctat ctggaaagag 2700
   agcaaggtga aaaaattatt caggagttcc tgtccaaggt gaaacaaatg ccctttactg 2760
   aaatgtcaga agaaaacatc acaataaagt taaaacagct aaaagctgaa gtaatagcaa 2820
   agaataatag ctttgtaaat gaaatcattt cacgaataaa agttactacg tgaaaaatcc 2880
   cagtaatgga atgaaggtaa tattgataag ctattgtctg taatagtttt atattgtttt 2940
   atattaaccc tttttccata gtgttaactg tcagtgccca tgggctatca acttaataag 3000
   atatttagta atattttact ttgaggacat tttcaaagat ttttattttg aaaaatgaga 3060
   gctgtaactg aggactgttt gcaattgaca taggcaataa taagtgatgt gctgaatttt 3120
   ataaataaaa tcatgtagtt tgtgg 3145
<210> 13
   <211> 756
   <212> PRT
   <213> Homo sapiens
<400> 13
   Met Ser Phe Val Ala Gly Val Phe Arg Arg Leu Asp Glu Thr Val Val
   1               5                   10                  15
   Asn Arg Phe Ala Ala Gly Glu Val Phe Gln Arg Pro Ala Asn Ala Phe
               20                  25                  30
   Lys Glu Met Phe Glu Asn Cys Leu Asp Ala Lys Ser Thr Ser Phe Gln
           35                  40                  45
   Val Phe Val Lys Glu Gly Gly Leu Lys Leu Phe Gln Phe Gln Asp Asn
       50                  55                  60
   Gly Thr Gly Phe Arg Lys Glu Asp Leu Asp Phe Val Cys Glu Arg Phe
   65                  70                  75                  80
   Thr Thr Ser Lys Leu Gln Ser Phe Glu Asp Leu Ala Ser Phe Ser Thr
                   85                  90                  95
   Tyr Gly Phe Arg Gly Glu Ala Leu Ala Ser Phe Ser His Val Ala His
               100                 105                 110
   Val Thr Phe Thr Thr Lys Thr Ala Asp Gly Lys Cys Ala Tyr Arg Ala
           115                 120                 125
   Ser Tyr Ser Asp Gly Lys Leu Lys Ala Pro Pro Lys Pro Cys Ala Gly
       130                 135                 140
   Asn Gln Gly Thr Gln Phe Thr Val Glu Asp Leu Phe Tyr Asn Phe Ala
   145                 150                 155                 160
   Thr Arg Arg Lys Ala Leu Lys Asn Pro Ser Glu Glu Tyr Gly Lys Phe
                   165                 170                 175
   Leu Glu Val Val Gly Arg Tyr Ser Val His Asn Ala Gly Phe Ser Phe
               180                 185                 190
   Ser Val Lys Lys Gln Gly Glu Thr Val Ala Asp Val Arg Thr Leu Pro
           195                 200                 205
   Asn Ala Ser Thr Val Asp Asn Ile Arg Ser Ile Phe Gly Asn Ala Val
       210                 215                 220
   Ser Arg Glu Leu Ile Glu Ile Gly Cys Glu Asp Lys Thr Leu Ala Phe
   225                 230                 235                 240
   Lys Met Asn Gly Tyr Ile Ser Asn Ala Asn Tyr Ser Val Lys Lys Cys
                   245                 250                 255
   Phe Phe Leu Leu Phe Phe Asn His Arg Leu Val Glu Ser Thr Ser Leu
               260                 265                 270
   Arg Lys Ala Ile Glu Thr Val Tyr Ala Ala Tyr Leu Pro Lys Asn Thr
           275                 280                 285
   His Pro Phe Leu Tyr Leu Ser Leu Glu Phe Ser Pro Gln Asn Val Asp
       290                 295                 300
   Val Asn Val His Pro Thr Lys His Glu Val His Phe Leu His Glu Glu
   305                 310                 315                 320
   Ser Phe Leu Glu Arg Val Gln Gln His Phe Glu Ser Lys Leu Leu Gly
                   325                 330                 335
   Ser Asn Ser Ser Arg Met Tyr Phe Thr Gln Thr Leu Leu Pro Gly Leu
               340                 345                 350
   Ala Gly Pro Ser Gly Glu Met Val Lys Ser Thr Thr Ser Leu Thr Ser
           355                 360                 365
   Ser Ser Thr Ser Gly Ser Ser Asp Lys Val Tyr Ala His Gln Met Val
   370 375 380
   Arg Thr Asp Ser Arg Glu Gln Lys Leu Asp Ala Phe Leu Gln Pro Leu
   385 390 395 400
   Ser Lys Pro Leu Ser Ser Gln Pro Gln Ala Phe Val Thr Glu Asp Lys
   405 410 415
   Thr Asp Phe Ser Ser Gly Arg Ala Arg Gln Gln Asp Glu Glu Met Leu
   420 425 430
   Glu Leu Pro Ala Pro Ala Glu Val Ala Ala Lys Asn Gln Ser Leu Glu
   435 440 445
   Gly Asp Thr Thr Lys Gly Thr Ser Glu Met Ser Glu Lys Arg Gly Pro
   450 455 460
   Thr Ser Ser Asn Pro Arg Lys Arg His Arg Glu Asp Ser Asp Val Glu
   465 470 475 480
   Met Val Glu Asp Asp Ser Arg Lys Glu Met Thr Ala Ala Cys Thr Pro
   485 490 495
   Arg Arg Arg Ile Ile Asn Leu Thr Ser Val Leu Ser Leu Gln Glu Glu
   500 505 510
   Ile Asn Glu Gln Gly His Glu Val Leu Arg Glu Met Leu His Asn His
   515 520 525
   Ser Phe Val Gly Cys Val Asn Pro Gln Trp Ala Leu Ala Gln His Gln
   530 535 540
   Thr Lys Leu Tyr Leu Leu Asn Thr Thr Lys Leu Ser Glu Glu Leu Phe
   545 550 555 560
   Tyr Gln Ile Leu Ile Tyr Asp Phe Ala Asn Phe Gly Val Leu Arg Leu
   565 570 575
   Ser Glu Pro Ala Pro Leu Phe Asp Leu Ala Met Leu Ala Leu Asp Ser
   580 585 590
   Pro Glu Ser Gly Trp Thr Glu Glu Asp Gly Pro Lys Glu Gly Leu Ala
   595 600 605
   Glu Tyr Ile Val Glu Phe Leu Lys Lys Lys Ala Glu Met Leu Ala Asp
   610 615 620
   Tyr Phe Ser Leu Glu Ile Asp Glu Glu Gly Asn Leu Ile Gly Leu Pro
   625                 630                 635                 640
   Leu Leu Ile Asp Asn Tyr Val Pro Pro Leu Glu Gly Leu Pro Ile Phe
                   645                 650                 655
   Ile Leu Arg Leu Ala Thr Glu Val Asn Trp Asp Glu Glu Lys Glu Cys
               660                 665                 670
   Phe Glu Ser Leu Ser Lys Glu Cys Ala Met Phe Tyr Ser Ile Arg Lys
           675                 680                 685
   Gln Tyr Ile Ser Glu Glu Ser Thr Leu Ser Gly Gln Gln Ser Glu Val
       690                 695                 700
   Pro Gly Ser Ile Pro Asn Ser Trp Lys Trp Thr Val Glu His Ile Val
   705                 710                 715                 720
   Tyr Lys Ala Leu Arg Ser His Ile Leu Pro Pro Lys His Phe Thr Glu
                   725                 730                 735
   Asp Gly Asn Ile Leu Gln Leu Ala Asn Leu Pro Asp Leu Tyr Lys Val
               740                 745                 750
   Phe Glu Arg Cys
           755
<210> 14
   <211> 2484
   <212> DNA
   <213> Homo sapiens
<400> 14
   cttggctctt ctggcgccaa aatgtcgttc gtggcagggg ttattcggcg gctggacgag 60
   acagtggtga accgcatcgc ggcgggggaa gttatccagc ggccagctaa tgctatcaaa 120
   gagatgattg agaactgttt agatgcaaaa tccacaagta ttcaagtgat tgttaaagag 180
   ggaggcctga agttgattca gatccaagac aatggcaccg ggatcaggaa agaagatctg 240
   gatattgtat gtgaaaggtt cactactagt aaactgcagt cctttgagga tttagccagt 300
   atttctacct atggctttcg aggtgaggct ttggccagca taagccatgt ggctcatgtt 360
   actattacaa cgaaaacagc tgatggaaag tgtgcataca gagcaagtta ctcagatgga 420
   aaactgaaag cccctcctaa accatgtgct ggcaatcaag ggacccagat cacggtggag 480
   gacctttttt acaacatagc cacgaggaga aaagctttaa aaaatccaag tgaagaatat
   540
   gggaaaattt tggaagttgt tggcaggtat tcagtacaca atgcaggcat tagtttctca
   600
   gttaaaaaac aaggagagac agtagctgat gttaggacac tacccaatgc ctcaaccgtg
   660
   gacaatattc gctccatctt tggaaatgct gttagtcgag aactgataga aattggatgt
   720
   gaggataaaa ccctagcctt caaaatgaat ggttacatat ccaatgcaaa ctactcagtg
   780
   aagaagtgca tcttcttact cttcatcaac catcgtctgg tagaatcaac ttccttgaga
   840
   aaagccatag aaacagtgta tgcagcctat ttgcccaaaa acacacaccc attcctgtac
   900
   ctcagtttag aaatcagtcc ccagaatgtg gatgttaatg tgcaccccac aaagcatgaa
   960
   gttcacttcc tgcacgagga gagcatcctg gagcgggtgc agcagcacat cgagagcaag
   1020
   ctcctgggct ccaattcctc caggatgtac ttcacccaga ctttgctacc aggacttgct
   1080
   ggcccctctg gggagatggt taaatccaca acaagtctga cctcgtcttc tacttctgga
   1140
   agtagtgata aggtctatgc ccaccagatg gttcgtacag attcccggga acagaagctt
   1200
   gatgcatttc tgcagcctct gagcaaaccc ctgtccagtc agccccaggc cattgtcaca
   1260
   gaggataaga cagatatttc tagtggcagg gctaggcagc aagatgagga gatgcttgaa
   1320
   ctcccagccc ctgctgaagt ggctgccaaa aatcagagct tggaggggga tacaacaaag
   1380
   gggacttcag aaatgtcaga gaagagagga cctacttcca gcaaccccag aaagagacat
   1440
   cgggaagatt ctgatgtgga aatggtggaa gatgattccc gaaaggaaat gactgcagct
   1500
   tgtacccccc ggagaaggat cattaacctc actagtgttt tgagtctcca ggaagaaatt
   1560
   aatgagcagg gacatgaggt tctccgggag atgttgcata accactcctt cgtgggctgt
   1620
   gtgaatcctc agtgggcctt ggcacagcat caaaccaagt tataccttct caacaccacc
   1680
   aagcttagtg aagaactgtt ctaccagata ctcatttatg attttgccaa ttttggtgtt
   1740
   ctcaggttat cggagccagc accgctcttt gaccttgcca tgcttgcctt agatagtcca
   1800
   gagagtggct ggacagagga agatggtccc aaagaaggac ttgctgaata cattgttgag
   1860
   tttctgaaga agaaggctga gatgcttgca gactatttct ctttggaaat tgatgaggaa
   1920
   gggaacctga ttggattacc ccttctgatt gacaactatg tgcccccttt ggagggactg
   1980
   cctatcttca ttcttcgact agccactgag gtgaattggg acgaagaaaa ggaatgtttt
   2040
   gaaagcctca gtaaagaatg cgctatgttc tattccatcc ggaagcagta catatctgag
   2100
   gagtcgaccc tctcaggcca gcagagtgaa gtgcctggct ccattccaaa ctcctggaag
   2160
   tggactgtgg aacacattgt ctataaagcc ttgcgctcac acattctgcc tcctaaacat
   2220
   ttcacagaag atggaaatat cctgcagctt gctaacctgc ctgatctata caaagtcttt
   2280
   gagaggtgtt aaatatggtt atttatgcac tgtgggatgt gttcttcttt ctctgtattc
   2340
   cgatacaaag tgttgtatca aagtgtgata tacaaagtgt accaacataa gtgttggtag
   2400
   cacttaagac ttatacttgc cttctgatag tattccttta tacacagtgg attgattata
   2460
   aataaataga tgtgtcttaa cata
   2484
<210> 15
   <211> 133
   <212> PRT
   <213> Homo sapiens
<400> 15
   Met Glu Arg Ala Glu Ser Ser Ser Thr Glu Pro Ala Lys Ala Ile Lys
   1               5                   10                  15
   Pro Ile Asp Arg Lys Ser Val His Gln Ile Cys Ser Gly Gln Val Val
               20                  25                  30
   Leu Ser Leu Ser Thr Ala Val Lys Glu Leu Val Glu Asn Ser Leu Asp
           35                  40                  45
   Ala Gly Ala Thr Asn Ile Asp Leu Lys Leu Lys Asp Tyr Gly Val Asp
       50                  55                  60
   Leu Ile Glu Val Ser Asp Asn Gly Cys Gly Val Glu Glu Glu Asn Phe
   65                  70                  75                  80
   Glu Gly Leu Thr Leu Lys His His Thr Ser Lys Ile Gln Glu Phe Ala
                   85                  90                  95
   Asp Leu Thr Gln Val Glu Thr Phe Gly Phe Arg Gly Glu Ala Leu Ser
               100                 105                 110
   Ser Leu Cys Ala Leu Ser Asp Val Thr Ile Ser Thr Cys His Ala Ser
           115                 120                 125
   Ala Lys Val Gly Thr
       130
<210> 16
   <211> 426
   <212> DNA
   <213> Homo sapiens
<400> 16
   cgaggcggat cgggtgttgc atccatggag cgagctgaga gctcgagtac agaacctgct
   60
   aaggccatca aacctattga tcggaagtca gtccatcaga tttgctctgg gcaggtggta
   120
   ctgagtctaa gcactgcggt aaaggagtta gtagaaaaca gtctggatgc tggtgccact
   180
   aatattgatc taaagcttaa ggactatgga gtggatctta ttgaagtttc agacaatgga
   240
   tgtggggtag aagaagaaaa cttcgaaggc ttaactctga aacatcacac atctaagatt
   300
   caagagtttg ccgacctaac tcaggttgaa acttttggct ttcgggggga agctctgagc
   360
   tcactttgtg cactgagcga tgtcaccatt tctacctgcc acgcatcggc gaaggttgga
   420
   acttga
   426
<210> 17
   <211> 19
   <212> DNA
   <213> Artificial
<220>
   <223> Oligonucleotide primer
<400> 17
   tttcgcaacg ggtttgccg
   19
<210> 18
   <211> 20
   <212> DNA
   <213> Artificial
<220>
   <223> Oligonucleotide primer
<400> 18
   gtttcagagt taagccttcg
   20
<210> 19
   <211> 13
   <212> DNA
   <213> Human immunoglobulin E light chain
<220>
   <221> misc_feature
   <222> (6)..(6)
   <223> n is a, c, g, or t
<400> 19
   tacgtngaat aat
   13
<210> 20
   <211> 13
   <212> DNA
   <213> Artificial
<220>
   <223> Consensus Sequence
<400> 20
   tacgttgaat aat
   13
<210> 21
   <211> 13
   <212> DNA
   <213> Human immunoglobulin E light chain
<400> 21
   tacgttgaat aat
   13
<210> 22
   <211> 63
   <212> DNA
   <213> Human immunoglobulin E light chain
<400> 22
   gttggatgtc ctatgaatca agggtttgag ggaagcgcct gacttctgct ggtaccagtg
   60
   caa
   63
<210> 23
   <211> 63
   <212> DNA
   <213> Human immunoglobulin E light chain
<400> 23
   gttggatgtc ctatgaatca agggtttggg ggaagcgcct gacttctgct ggtaccagtg 60
   caa 63
<210> 24
   <211> 63
   <212> DNA
   <213> Artificial
<220>
   <223> Consensus Sequence
<400> 24
   gttggatgtc ctatgaatca agggtttgrg ggaagcgcct gacttctgct ggtaccagtg 60
   caa 63

## Description

### TECHNICAL FIELD OF THE INVENTION

The invention is related to the area of antibody maturation and cellular production. In particular, it is related to the field of mutagenesis.

### BACKGROUND OF THE INVENTION

The use of antibodies to block the activity of foreign and/or endogenous polypeptides provides an effective and selective strategy for treating the underlying cause of disease. In particular is the use of monoclonal antibodies (MAb) as effective therapeutics such as the FDA approved ReoPro (Glaser, V. (1996) Can ReoPro repolish tarnished monoclonal therapeutics? Nat. Biotechnol. 14:1216-1217), an anti-platelet MAb from Centocor; Herceptin (Weiner, L.M. (1999) Monoclonal antibody therapy of cancer. Semin. Oncol. 26:43-51), an anti-Her2/neu MAb from Genentech; and Synagis (Saez-Llorens, X.E., et al. (1998) Safety and pharmacokinetics of an intramuscular humanized monoclonal antibody to respiratory syncytial virus in premature infants and infants with bronchopulmonary dysplasia. Pediat. Infect. Dis. J. 17:787-791), an anti-respiratory syncytial virus MAb produced by Medimmune.

Standard methods for generating MAbs against candidate protein targets are known by those skilled in the art. Briefly, rodents such as mice or rats are injected with a purified antigen in the presence of adjuvant to generate an immune response (Shield, C.F., et al. (1996) A cost-effective analysis of OKT3 induction therapy in cadaveric kidney transplantation. Am. J. Kidney Dis. 27:855-864). Rodents with positive immune sera are sacrificed and splenocytes are isolated. Isolated splenocytes are fused to melanomas to produce immortalized cell lines that are then screened for antibody production. Positive lines are isolated and characterized for antibody production. The direct use of rodent MAbs as human therapeutic agents were confounded by the fact that human anti-rodent antibody (HARA) responses occurred in a significant number of patients treated with the rodent-derived antibody (Khazaeli, M.B., et al., (1994) Human immune response to monoclonal antibodies. J. Immunother. 15:42-52). In order to circumvent the problem of HARA, the grafting of the complementarity determining regions (CDRs), which are the critical motifs found within the heavy and light chain variable regions of the immunoglobulin (Ig) subunits making up the antigen binding domain, onto a human antibody backbone found these chimeric molecules are able to retain their binding activity to antigen while lacking the HARA response (Emery, S.C., and Harris, W.J. "Strategies for humanizing antibodies" In: ANTIBODY ENGINEERING C.A.K. Borrebaeck (Ed.) Oxford University Press, N.Y. 1995. pp. 159-183. A common problem that exists during the "humanization" of rodent-derived MAbs (referred to hereon as HAb) is the loss of binding affinity due to conformational changes in the 3 dimensional structure of the CDR domain upon grafting onto the human Ig backbone (U.S. Patent No. 5,530,101 to Queen et al.). To overcome this problem, additional HAb vectors are usually needed to be engineeredby inserting or deleting additional amino acid residues within the framework region and/or within the CDR coding region itself in order to recreate high affinity HAbs (U.S. Patent No. 5,530,101 to Queen et al.). This process is a very time consuming procedure that involves the use of expensive computer modeling programs to predict changes that may lead to a high affinity HAb. In some instances the affinity of the HAb is never restored to that of the MAb, rendering them of little therapeutic use.

Another problem that exists in antibody engineering is the generation of stable, high yielding producer cell lines that is required for manufacturing of the molecule for clinical materials. Several strategies have been adopted in standard practice by those skilled in the art to circumvent this problem. One method is the use of Chinese Hamster Ovary (CHO) cells transfected with exogenous Ig fusion genes containing the grafted human light and heavy chains to produce whole antibodies or single chain antibodies, which are a chimeric molecule containing both light and heavy chains that form an antigen-binding polypeptide (Reff, M.E. (1993) High-level production of recombinant immunoglobulins in mammalian cells. Curr. Opin. Biotechnol. 4:573-576). Another method employs the use of human lymphocytes derived from transgenic mice containing a human grafted immune system or transgenic mice containing a human Ig gene repertoire. Yet another method employs the use of monkeys to produce primate MAbs, which have been reported to lack a human anti-monkey response (Neuberger, M., and Gruggermann, M. (1997) Monoclonal antibodies. Mice perform a human repertoire. Nature 386:25-26). In all cases, the generation of a cell line that is capable of generating sufficient amounts of high affinity antibody poses a major limitation for producing sufficient materials for clinical studies. Because of these limitations, the utility of other recombinant systems such as plants are currently being explored as systems that will lead to the stable, high-level production of humanized antibodies (Fiedler, U., and Conrad, U. (1995) High-level production and long-term storage of engineered antibodies in transgenic tobacco seeds. Bio/Technology 13:1090-1093).

Irving, R.A. et al; Immunotechnology 2 (1996) 127 to 143 discloses a method for providing genetically altered antibodies by the recombinant expression of a library of mutant antibodies in E.coli mutator cells.

CA 2,240,609 discloses a method of generating hypermutable cells and organisms by the introduction of dominant negative alleles of human mismatch repair genes.

A method for generating diverse antibody sequences within the variable domain that results in HAbs and Mabs with high binding affinities to antigens would be useful for the creation of more potent therapeutic and diagnostic reagents respectively. Moreover, the generation of randomly altered nucleotide and polypeptide residues throughout an entire antibody molecule will result in new reagents that are less antigenic and/or have beneficial pharmacokinetic properties. The invention described herein is directed to the use of random genetic mutation throughout an antibody structure *in vivo* by blocking the endogenous mismatch repair (MMR) activity of a host cell producing immunoglobulins that encode biochemically active antibodies. The invention also relates to methods for repeated *in vivo* genetic alterations and selection for antibodies with enhanced binding and pharmacokinetic profiles.

In addition, the ability to develop genetically altered host cells that are capable of secreting increased amounts of antibody will also provide a valuable method for creating cell hosts for product development. The invention described herein is direct to the creation of genetically altered cell hosts with increased antibody production via the blockade of MMR.

The invention facilitates the generation of high affinity antibodies and the production of cell lines with elevated levels of antibody production. Other advantages of the present invention are described in the examples and figures described herein.

### SUMMARY OF THE INVENTION

The invention provides a method of making a hypermutable, antibody-producing cell, comprising introducing into a cell capable of producing antibodies a polynucleotide comprising a dominant negative allele of a mismatch repair gene, wherein said mismatch repair gene is PMS2.

The invention also provides a method of generating a mutation in an antibody-producing cell, the mutation causing over expression, enhanced secretion or enhanced affinity for antigen of an antibody produced by the cell, the method comprising:
growing said antibody-producing cell comprising a dominant negative allele of a mismatch repair gene; and
testing for the over expression, enhanced secretion or enhanced affinity for antigen of an antibody produced by the cell,
   wherein said mismatch repair gene is PMS2.

The invention further provides a method of reversibly altering the hypermutability of an antibody-producing cell, comprising introducing an inducible vector into a cell, wherein said inducible vector comprises a dominant negative allele of a mismatch repair gene operably linked to an inducible promoter, and inducing said cell to express said dominant negative mismatch repair gene; wherein said mismatch repair gene is PMS2.
The invention yet further provides a method of producing genetically altered antibodies, comprising
transfecting a polynucleotide encoding an immunoglobulin protein into a cell, wherein said cell comprises a dominant negative mismatch repair gene;
growing said cell, thereby producing a hypermutated polynucleotide encoding a hypermutated immunoglobulin protein;
screening for a desirable property of said hypermutated immunoglobulin protein;
isolating said hypermutated polynucleotide; and
transfecting said hypermutated polynucleotide into a genetically stable cell, thereby producing a hypermutated antibody-producing, genetically stable cell;
   wherein said mismatch repair gene is PMS2.
The methods of the invention may further comprise the step of restoring the genetic stability of said cells.

The invention yet further provides a hypermutated antibody-producing cell comprising a polynucleotide encoding an antibody and a polynucleotide encoding a dominant negative allele of PMS2. The hypermutated antibody-producing cells of the invention may optionally be genetically stable.

The invention further provides homogeneous cultures of cells of the invention.

The antibody producing cells suitable for use in the invention include, but are not limited to rodent, primate, or human hybridomas or lymphoblastoids; mammalian cells transfected and expressing exogenous Ig subunits or chimeric single chain molecules; plant cells, yeast or bacteria transfected and expressing exogenous Ig subunits or chimeric single chain molecules.

The cells that are capable of producing antibodies for use in the present invention include cells that naturally produce antibodies, and cells that are engineered to produce antibodies through the introduction of immunoglobulin encoding sequences. Conveniently, the introduction of polynucleotide sequences into cells is accomplished by transfection.

The dominant negative allele of a *PMS2* may be a truncation mutation (preferably a truncation mutation at codon 134, or a thymidine at nucleotide 424 of wild-type *PMS2*).

These and other aspects of the invention are provided by one or more of the embodiments described below. In one embodiment of the invention, a method for making an antibody producing cell line hypermutable is provided. A polynucleotide encoding a dominant negative allele of a MMR gene is introduced into an antibody-producing cell. The cell becomes hypermutable as a result of the introduction of the gene.

In another embodiment of the invention, a method is provided for introducing a mutation into an endogenous gene encoding for an immunoglobulin polypeptide or a single chain antibody. A polynucleotide encoding a dominant negative allele of a MMR gene is introduced into a cell. The cell becomes hypermutable as a result of the introduction and expression of the MMR gene allele. The cell further comprises an immunoglobulin gene of interest. The cell is grown and tested to determine whether the gene encoding for an immunoglobulin or a single chain antibody of interest harbors a mutation. In another aspect of the invention, the gene encoding the mutated immunoglobulin polypeptide or single chain antibody may be isolated and expressed in a genetically stable cell. In a preferred embodiment, the mutated antibody is screened for at least one desirable property such as, but not limited to, enhanced binding characteristics.

In another embodiment of the invention, a gene or set of genes encoding for Ig light and heavy chains or a combination therein are introduced into a mammalian cell host that is MMR defective. The cell is grown, and clones are analyzed for antibodies with enhanced binding characteristics.

In another embodiment of the invention, a method will be provided for producing new phenotypes of a cell. A polynucleotide encoding a dominant negative allele of a MMR gene is introduced into a cell. The cell becomes hypermutable as a result of the introduction of the gene. The cell is grown. The cell is tested for the expression of new phenotypes where the phenotype is enhanced secretion of a polypeptide.

These and other embodiments of the invention provide the art with methods that can generate enhanced mutability in cells as well as providing cells harboring potentially useful mutations for the large-scale production of high affinity antibodies with beneficial pharmacokinetic profiles.

### BRIEF DESCRIPTION OF THE DRAWINGS

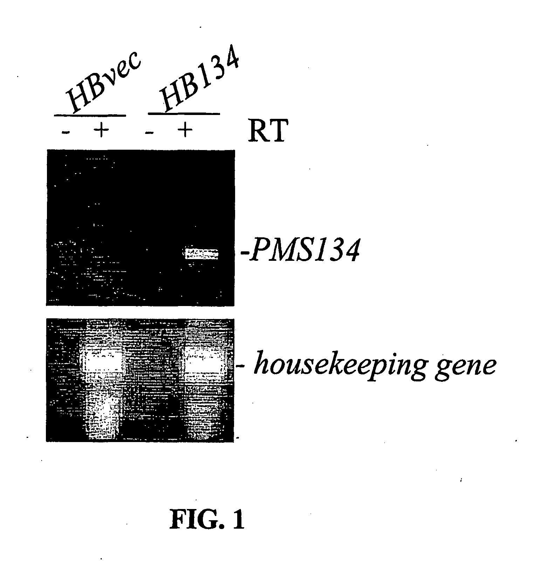

**Figure 1****.** Hybridoma cells stably expressing PMS2 and PMS134 MMR genes. Shown is steady state mRNA expression of MMR genes transfected into a murine hybridoma cell line. Stable expression was found after 3 months of continuous growth. The (-) lanes represent negative controls where no reverse transcriptase was added, and the (+) lanes represent samples reverse transcribed and PCR amplified for the MMR genes and an internal housekeeping gene as a control.

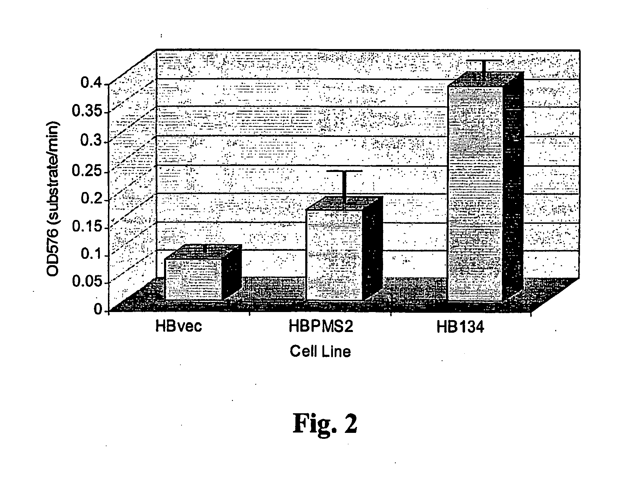

**Figure 2****.** Creation of genetically hypermutable hybridoma cells. Dominant negative MMR gene alleles were expressed in cells expressing a MMR-sensitive reporter gene. Dominant negative alleles such as PMS134 and the expression of MMR genes from other species results in antibody producer cells with a hypermutable phenotype that can be used to produce genetically altered immunoglobulin genes with enhanced biochemical features as well as lines with increased Ig expression and/or secretion. Values shown represent the amount of converted CPRG substrate which is reflective of the amount of function □-galactosidase contained within the cell from genetic alterations within the pCAR-OF reporter gene. Higher amounts of β-galactosidase activity reflect a higher mutation rate due to defective MMR.

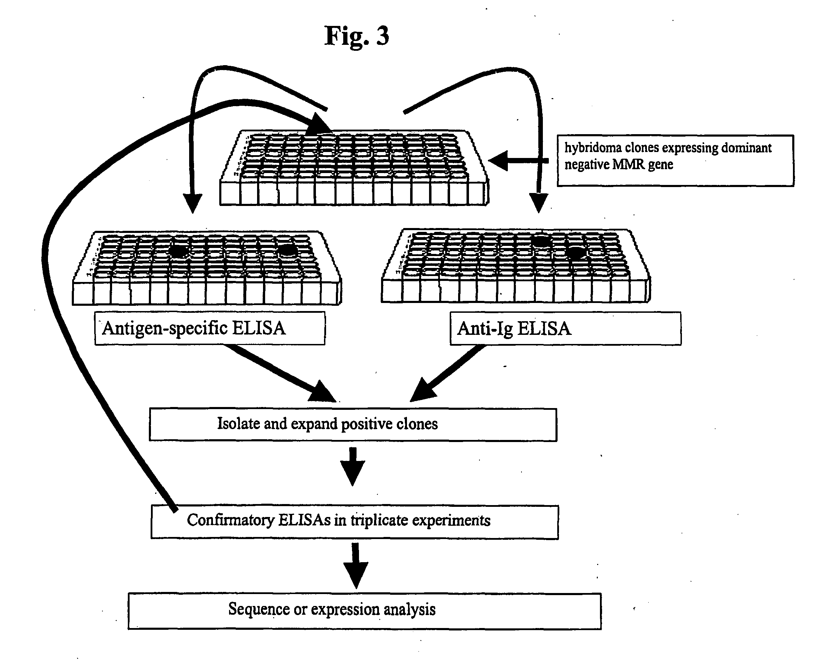

**Figure 3****.** Screening method for identifying antibody-producing cells containing antibodies with increased binding activity and/or increased expression/secretion.

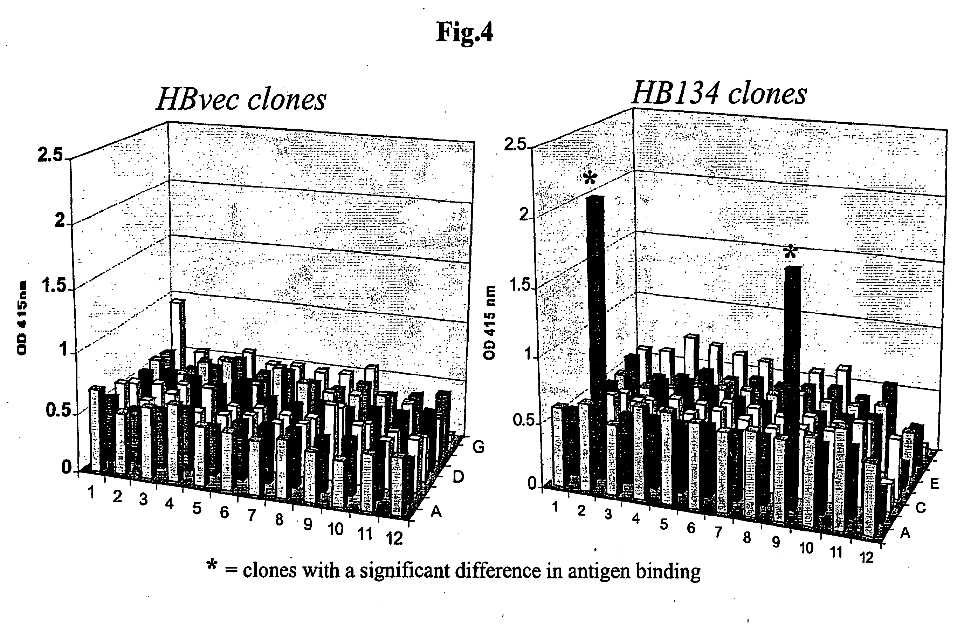

**Figure 4****.** Generation of a genetically altered antibody with an increased binding activity. Shown are ELISA values from 96-well plates, screened for antibodies specific to hIgE. Two clones with a high binding value were found in HB134 cultures.

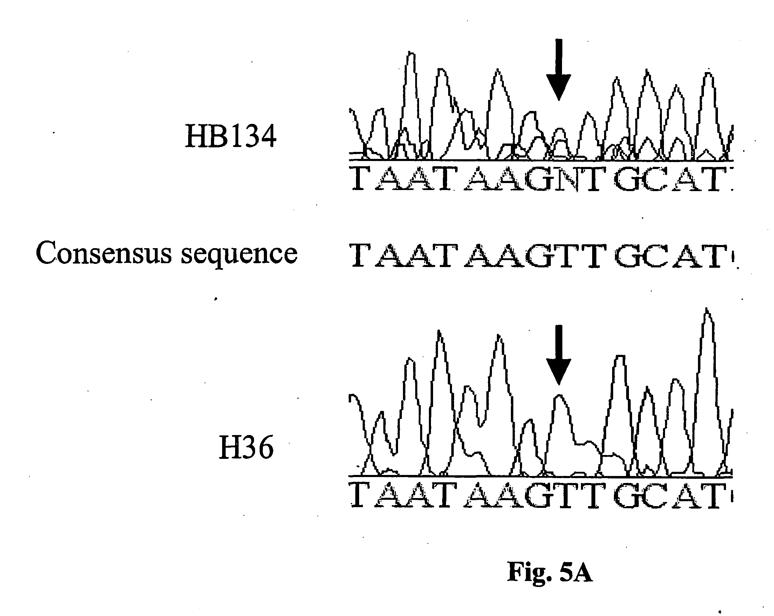

**Figure 5A**. Sequence alteration within variable chain of an antibody (a mutation within the light chain variable region in MMR-defective HB134 antibody producer cells). Arrows indicate the nucleotide at which a mutation occurred in a subset of cells from a clone derived from HB134 cells. The HB134 sequence (SEQ ID NO:19) is shown as the top line, the consensus sequence (SEQ ID NO:20) as the middle line, and the parental H36 sequence (SEQ ID NO:21) is shown below the sequence tracing. The change results in a Thr to Ser change within the light chain variable region. The coding sequence is in the antisense direction.

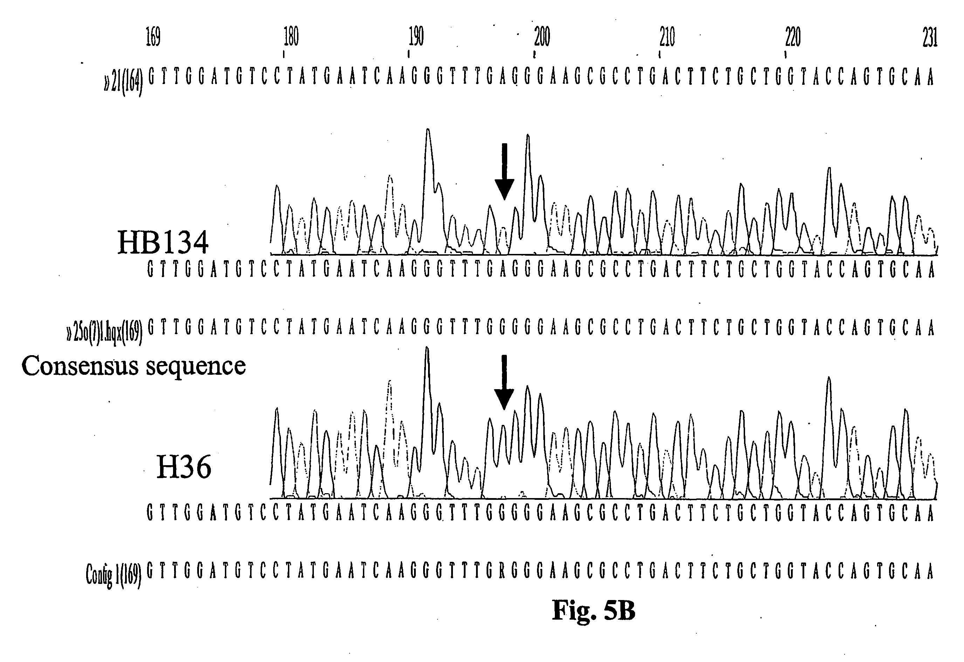

**Figure 5B****.** Sequence alteration within variable chain of an antibody (a mutation within the light chain variable region in MMR-defective HB134 antibody producer cells). The HB134 sequence (SEQ ID NO:22) is shown above and below the tracing for the HB134 sequence, and the parental H36 sequence (SEQ ID NO:23) is shown above and below the H36 sequence tracing. A consensus sequence (SEQ ID NO:24) is shown at the bottom of the figure. Arrows indicate the nucleotide at which a mutation occurred in a subset of cells from a clone derived from HB134 cells. The change results in a Pro to His Leu change within the light chain variable region.

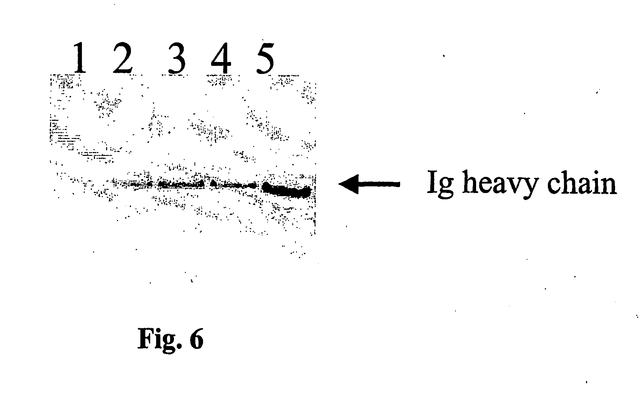

**Figure 6****.** Generation of MMR-defective clones with enhanced steady state Ig protein levels. A Western blot of heavy chain immunglobulins from HB134 clones with high levels of MAb (>500ngs/ml) within the conditioned medium shows that a subset of clones express higher steady state levels of immunoglobulins (Ig). The H36 cell line was used as a control to measure steady state levels in the parental strain. Lane 1: fibroblast cells (negative control); Lane 2: H36 cell; Lane 3: HB134 clone with elevated MAb levels; Lane 4: HB134 clone with elevated MAb levels; Lane 5: HB134 clone with elevated MAb levels.

Methods have been discovered for developing hypermutable antibody-producing cells by taking advantage of the conserved mismatch repair (MMR) process of host cells. Dominant negative alleles of such genes, when introduced into cells or transgenic animals, increase the rate of spontaneous mutations by reducing the effectiveness of DNA repair and thereby render the cells or animals hypermutable. Hypermutable cells or animals can then be utilized to develop new mutations in a gene of interest. Blocking MMR in antibody-producing cells such as but not limited to: hybridomas; mammalian cells transfected with genes encoding for Ig light and heavy chains; mammalian cells transfected with genes encoding for single chain antibodies; eukaryotic cells transfected with Ig genes, can enhance the rate of mutation within these cells leading to clones that have enhanced antibody production and/or cells containing genetically altered antibodies with enhanced biochemical properties such as increased antigen binding. The process of MMR, also called mismatch proofreading, is carried out by protein complexes in cells ranging from bacteria to mammalian cells. A MMR gene is a gene that encodes for one of the proteins of such a mismatch repair complex. Although not wanting to be bound by any particular theory of mechanism of action, a MMR complex is believed to detect distortions of the DNA helix resulting from non-complementary pairing of nucleotide bases. The non-complementary base on the newer DNA strand is excised, and the excised base is replaced with the appropriate base, which is complementary to the older DNA strand. In this way, cells eliminate many mutations that occur as a result of mistakes in DNA replication.

Dominant negative alleles cause a MMR defective phenotype even in the presence of a wild-type allele in the same cell. An example of a dominant negative allele of a MMR gene is the human gene *hPMS2-134*, which carries a truncating mutation at codon 134 (SEQ ID NO:15). The mutation causes the product of this gene to abnormally terminate at the position of the 134th amino acid, resulting in a shortened polypeptide containing the N-terminal 133 amino acids. Such a mutation causes an increase in the rate of mutations, which accumulate in cells after DNA replication. Expression of a dominant negative allele of a mismatch repair gene results in impairment of mismatch repair activity, even in the presence of the wild-type allele. Any allele of *PMS2* which produces such effect can be used in this invention. Dominant negative alleles of a MMR gene can be obtained from the cells of humans, animals, yeast, bacteria, or other organisms. Such alleles can be identified by screening cells for defective MMR activity. Cells from animals or humans with cancer can be screened for defective mismatch repair. Cells from colon cancer patients may be particularly useful. Genomic DNA, cDNA, or mRNA from any cell encoding a MMR protein can be analyzed for variations from the wild type sequence. Dominant negative alleles of a MMR gene can also be created artificially, for example, by producing variants of the *hPMS2-134* allele or other MMR genes. Various techniques of site-directed mutagenesis can be used. The suitability of such alleles, whether natural or artificial, for use in generating hypermutable cells or animals can be evaluated by testing the mismatch repair activity caused by the allele in the presence of one or more wild-type alleles, to determine if it is a dominant negative allele.

A cell or an animal into which a dominant negative allele of a mismatch repair gene has been introduced will become hypermutable. This means that the spontaneous mutation rate of such cells or animals is elevated compared to cells or animals without such alleles. The degree of elevation of the spontaneous mutation rate can be at least 2-fold, 5-fold, 10-fold, 20-fold, 50-fold, 100-fold, 200-fold, 500-fold or 1000-fold that of the normal cell or animal. The use of chemical mutagens such as but limited to methane sulfonate, dimethyl sulfonate, O6-methyl benzadine, MNU, ENU, etc. can be used in MMR defective cells to increase the rates an additional 10 to 100 fold that of the MMR deficiency itself.

According to one aspect of the invention, a polynucleotide encoding for a dominant negative form of a MMR protein is introduced into a cell. The gene can be any dominant negative allele of *PMS2*, which is part of a MMR complex. The dominant negative allele can be naturally occurring or made in the laboratory. The polynucleotide can be in the form of genomic DNA, cDNA, RNA, or a chemically synthesized polynucleotide.

The polynucleotide can be cloned into an expression vector containing a constitutively active promoter segment (such as but not limited to CMV, SV40, Elongation Factor or LTR sequences) or to inducible promoter sequences such as the steroid inducible pIND vector (Invitrogen), where the expression of the dominant negative MMR gene can be regulated. The polynucleotide can be introduced into the cell by transfection.

According to another aspect of the invention, an immunoglobulin (Ig) gene, a set of Ig genes or a chimeric gene containing whole or parts of an Ig gene can be transfected into MMR deficient cell hosts, the cell is grown and screened for clones containing genetically altered Ig genes with new biochemical features. MMR defective cells may be of human, primates, mammals, rodent, plant, yeast or of the prokaryotic kingdom. The mutated gene encoding the Ig with new biochemical features may be isolated from the respective clones and introduced into genetically stable cells (*i*.*e*., cells with normal MMR) to provide clones that consistently produce Ig with the new biochemical features. The method of isolating the Ig gene encoding Ig with new biochemical features may be any method known in the art. Introduction of the isolated polynucleotide encoding the Ig with new biochemical features may also be performed using any method known in the art, including, but not limited to transfection of an expression vector containing the polynucleotide encoding the Ig with new biochemical features. As an alternative to transfecting an Ig gene, a set of Ig genes or a chimeric gene containing whole or parts of an Ig gene into an MMR deficient host cell, such Ig genes may be transfected simultaneously with a gene encoding a dominant negative mismatch repair gene into a genetically stable cell to render the cell hypermutable.

Transfection is any process whereby a polynucleotide is introduced into a cell. The process of transfection can be carried out in a living animal, *e*.*g*., using a vector for gene therapy, or it can be carried out *in vitro*, *e*.*g*., using a suspension of one or more isolated cells in culture. The cell can be any type of eukaryotic cell, including, for example, cells isolated from humans or other primates, mammals or other vertebrates, invertebrates, and single celled organisms such as protozoa, yeast, or bacteria.

In general, transfection will be carried out using a suspension of cells, or a single cell, but other methods can also be applied as long as a sufficient fraction of the treated cells or tissue incorporates the polynucleotide so as to allow transfected cells to be grown and utilized. The protein product of the polynucleotide may be transiently or stably expressed in the cell. Techniques for transfection are well known. Available techniques for introducing polynucleotides include but are not limited to electroporation, transduction, cell fusion, the use of calcium chloride, and packaging of the polynucleotide together with lipid for fusion with the cells of interest. Once a cell has been transfected with the MMR gene, the cell can be grown and reproduced in culture. If the transfection is stable, such that the gene is expressed at a consistent level for many cell generations, then a cell line results.

An isolated cell is a cell obtained from a tissue of humans or animals by mechanically separating out individual cells and transferring them to a suitable cell culture medium, either with or without pretreatment of the tissue with enzymes, *e*.*g*., collagenase or trypsin. Such isolated cells are typically cultured in the absence of other types of cells. Cells selected for the introduction of a dominant negative allele of a mismatch repair gene may be derived from a eukaryotic organism in the form of a primary cell culture or an immortalized cell line, or may be derived from suspensions of single-celled organisms.

A polynucleotide encoding for a dominant negative form of a MMR protein can be introduced into the genome of an animal by producing a transgenic animal. The animal can be any species for which suitable techniques are available to produce transgenic animals. For example, transgenic animals can be prepared from domestic livestock, *e*.*g*., bovine, swine, sheep, goats, horses, etc.; from animals used for the production of recombinant proteins, *e*.*g*., bovine, swine, or goats that express a recombinant polypeptide in their milk; or experimental animals for research or product testing, *e*.*g*., mice, rats, guinea pigs, hamsters, rabbits, etc. Cell lines that are determined to be MMR defective can then be used as a source for producing genetically altered immunoglobulin genes *in vitro* by introducing whole, intact immunoglobulin genes and/or chimeric genes encoding for single chain antibodies into MMR defective cells from any tissue of the MMR defective animal.

Once a transfected cell line or a colony of transgenic animals has been produced, it can be used to generate new mutations in one or more gene(s) of interest. A gene of interest can be any gene naturally possessed by the cell line or transgenic animal or introduced into the cell line or transgenic animal. An advantage of using such cells or animals to induce mutations is that the cell or animal need not be exposed to mutagenic chemicals or radiation, which may have secondary harmful effects, both on the object of the exposure and on the workers. However, chemical mutagens may be used in combination with MMR deficiency, which renders such mutagens less toxic due to an undetermined mechanism. Hypermutable animals can then be bred and selected for those producing genetically variable B-cells that may be isolated and cloned to identify new cell lines that are useful for producing genetically variable cells. Once a new trait is identified, the dominant negative MMR gene allele can be removed by directly knocking out the allele by technologies used by those skilled in the art or by breeding to mates lacking the dominant negative allele to select for offspring with a desired trait and a stable genome. Another alternative is to use a CRE-LOX expression system, whereby the dominant negative allele is spliced from the animal genome once an animal containing a genetically diverse immunoglobulin profile has been established. Yet another alternative is the use of inducible vectors such as the steroid induced pIND (Invitrogen) or pMAM (Clonetech) vectors which express exogenous genes in the presence of corticosteroids.

Mutations can be detected by analyzing for alterations in the genotype of the cells or animals, for example by examining the sequence of genomic DNA, cDNA, messenger RNA, or amino acids associated with the gene of interest Mutations can also be detected by screening for the production of antibody titers. A mutant polypeptide can be detected by identifying alterations in electrophoretic mobility, spectroscopic properties, or other physical or structural characteristics of a protein encoded by a mutant gene. One can also screen for altered function of the protein *in situ*, in isolated form, or in model systems. One can screen for alteration of any property of the cell or animal associated with the function of the gene of interest, such as but not limited to Ig secretion.

Examples of mismatch repair proteins and nucleic acid sequences include the following:
PMS2 (mousse) (SEQ ID NO:5),
PMS2 (mouse cDNA) (SEQ ID NO:6),
PMS2 (human) (SEQ ID NO:7),
PMS2 (human cDNA) (SEQ ID NO:8),
PMS1 (human) (SEQ ID NO:9),
PMS1 (human) (SEQ ID NO: 10),
MSH2 (human) (SEQ ID NO:11), MSH2 (human cDNA) (SEQ ID NO:12), MLH1 (human) (SEQ ID NO:13),
MLH1 (human) (SEQ ID NO:14),
hPMS2-134 (human) (SEQ ID NO:15) and hPMS2-134 (human cDNA) (SEQ ID NO:16)

For further information on the background of the invention the following references may be consulted:
1. Glaser, V. (1996) Can ReoPro repolish tarnished monoclonal therapeutics? Nat. Biotechol. 14:1216-1217.
2. Weiner, L.M. (1999) Monoclonal antibody therapy of cancer. Semin. Oncol. 26:43-51.
3. Saez-Llorens, X.E. et al. (1998) Safety and pharmacokinetics of an intramuscular humanized monoclonal antibody to respiratory syncytial virus in premature infants and infants with bronchopulmonary dysplasia. Pediat. Infect Dis. J. 17:787-791.
4. Shield, C.F. et al. (1996) A cost-effective analysis of OKT3 induction therapy in cadaveric kidney transplantation. Am. J. Kidney Dis. 27:855-864.
5. Khazaeli, M.B. et al. (1994) Human immune response to monoclonal antibodies. J. Immunother. 15:42-52.
6. Emery, S.C. and W.J. Harris "Strategies for humanizing antibodies" In: ANTIBODY ENGINEERING C.A.K. Borrebaeck (Ed.) Oxford University Press, N.Y. 1995, pp. 159-183.
7. U.S. Patent No. 5,530,101to Queen and Selick.
8. Reff, M.E. (1993) High-level production of recombinant immunoglobulins in mammalian cells. Curr. Opin. Biotechnol. 4:573-576.
9. Neuberger, M. and M. Gruggermann, (1997) Monoclonal antibodies. Mice perform a human repertoire. Nature 386:25-26.
10. Fiedler, U. and U. Conrad (1995) High-level production and long-term storage of engineered antibodies in transgenic tobacco seeds. Bio/Technology 13:1090-1093.
11. Baker S.M. et al. (1995) Male defective in the DNA mismatch repair gene PMS2 exhibit abnormal chromosome synapsis in meiosis. Cell 82:309-319.
12. Bronner, C.E. et al. (1994) Mutation in the DNA mismatch repair gene homologue hMLH1 is associated with hereditary non-polyposis colon cancer. Nature 368:258-261.
13. de Wind N. et al. (1995) Inactivation of the mouse Msh2 gene results in mismatch repair deficiency, methylation tolerance, hyperrecombination, and predisposition to cancer. Cell 82:321-300.
14. Drummond, J.T. et al. (1995) Isolation of an hMSH2-p160 heterodimer that restores mismatch repair to tumor cells. Science 268:1909-1912.
15. Modrich, P. (1994) Mismatch repair, genetic stability, and cancer. Science 266:1959-1960.
16. Nicolaides, N.C. et al. (1998) A Naturally Occurring hPMS2 Mutation Can Confer a Dominant Negative Mutator Phenotype. Mol. Cell. Biol. 18:1635-1641.
17. Prolla, T.A. et al. (1994) MLH1, PMS1, and MSH2 Interaction during the initiation of DNA mismatch repair in yeast. Science 264:1091-1093.
18. Strand, M. et al. (1993) Destabilization of tracts of simple repetitive DNA in yeast by mutations affecting DNA mismatch repair. Nature 365:274-276.
19. Su, S.S., R.S. Lahue, K.G. Au, and P. Modrich (1988) Mispair specificity of methyl directed DNA mismatch corrections in vitro. J. Biol. Chem. 263:6829-6835.
20. Parsons, R. et al. (1993) Hypermutability and mismatch repair deficiency in RER+ tumor cells. Cell 75:1227-1236.
21. Papadopoulos, N. et al. (1993) Mutation of a mutL homolog is associated with hereditary colon cancer. Science 263:1625-1629.
22. Perucho, M. (1996) Cancer of the microsatellite mutator phenotype. Biol. Chem. 377:675-684.
23. Nicolaides N.C., K.W. Kinzler, and B. Vogelstein (1995) Analysis of the 5' region of PMS2 reveals heterogenous transcripts and a novel overlapping gene. Genomics 29:329-334.
24. Nicolaides, N.C. et al. (1995) Genomic organization of the human PMS2 gene family. Genomics 30:195-206.
25. Palombo, F. et al. (1994) Mismatch repair and cancer. Nature 36:417.
26. Eshleman J.R. and S.D. Markowitz (1996) Mismatch repair defects in human carcinogenesis. Hum. Mol. Genet. 5:1489-494.
27. Liu, T. et al. (2000) Microsatellite instability as a predictor of a mutation in a DNA mismatch repair gene in familial colorectal cancer. Genes Chromosomes Cancer 27:17-25.
28. Nicolaides, N.C. et al. (1992) The Jun family members, c-JUN and JUND, transactivate the human c-myb promoter via an Ap1 like element. J. Biol. Chem. 267:19665-19672.
29. Shields, R.L. et al. (1995) Anti-IgE monoclonal antibodies that inhibit allergen-specific histamine release. Int. Arch. Allergy Immunol. 107:412-413.
30. Frigerio L. et al. (2000) Assembly, secretion, and vacuolar delivery of a hybrid immunoglobulin in plants. Plant Physiol. 123:1483-1494.
31. Bignami M, (2000) Unmasking a killer: DNA O(6)-methylguanine and the cytotoxicity of methylating agents. Mutat. Res. 462:71-82.
32. Drummond, J.T. et al. (1996) Cisplatin and adriamycin resistance are associated with MutLa and mismatch repair deficiency in an ovarian tumor cell line. J. Biol. Chem. 271:9645-19648.
33. Galio, L. et al. (1999) ATP hydrolysis-dependent formation of a dynamic ternary nucleoprotein complex with MutS and MutL. Nucl. Acids Res. 27:2325-23231.

The above disclosure generally describes the present invention. A more complete understanding can be obtained by reference to the following specific examples which are provided herein for purposes of illustration only, and are not intended to limit the scope of the invention.

### EXAMPLE 1: Stable expression of dominant negative MMR genes in hybridoma cells

It has been previously shown by Nicolaides et al. (Nicolaides et al. (1998) A Naturally Occurring hPMS2 Mutation Can Confer a Dominant Negative Mutator Phenotype Mol. Cell. Biol. 18:1635-1641) that the expression of a dominant negative allele in an otherwise MMR proficient cell could render these host cells MMR deficient. The creation of MMR deficient cells can lead to the generation of genetic alterations throughout the entire genome of a host organisms offspring, yielding a population of genetically altered offspring or siblings that may produce biochemicals with altered properties. This patent application teaches of the use of dominant negative MMR genes in antibody-producing cells, including but not limited to rodent hybridomas, human hybridomas, chimeric rodent cells producing human immunoglobulin gene products, human cells expressing immunoglobulin genes, mammalian cells producing single chain antibodies, and prokaryotic cells producing mammalian immunoglobulin genes or chimeric immunoglobulin molecules such as those contained within single-chain antibodies. The cell expression systems described above that are used to produce antibodies are well known by those skilled in the art of antibody therapeutics.

To demonstrate the ability to create MMR defective hybridomas using dominant negative alleles of MMR genes, we first transfected a mouse hybridoma cell line that is known to produce and antibody directed against the human IgE protein with an expression vector containing the human PMS2 (cell line referred to as HBPMS2), the previously published dominant negative PMS2 mutant referred herein as PMS 134 (cell line referred to as HB134), or with no insert (cell line referred to as HBvec). The results showed that the PMS 134 mutant could indeed exert a robust dominant negative effect, resulting in biochemical and genetic manifestations of MMR deficiency. Unexpectedly was the finding that the full length PMS2 also resulted in a lower MMR activity while no effect was seen in cells containing the empty vector. A brief description of the methods is provided below.

The MMR proficient mouse H36 hybridoma cell line was transfected with various *hPMS2* expression plasmids plus reporter constructs for assessing MMR activity. The MMR genes were cloned into the pEF expression vector, which contains the elongation factor promoter upstream of the cloning site followed by a mammalian polyadenylation signal. This vector also contains the NEOr gene that allows for selection of cells retaining this plasmid. Briefly, cells were transfected with 1 µg of each vector using polyliposomes following the manufacturer's protocol (Life Technologies). Cells were then selected in 0.5 mg/ml of G418 for 10 days and G418 resistant cells were pooled together to analyze for gene expression. The pEF construct contains an intron that separates the exon 1 of the EF gene from exon 2, which is juxtaposed to the 5' end of the polylinker cloning site. This allows for a rapid reverse transcriptase polymerase chain reaction (RT-PCR) screen for cells expressing the spliced products. At day 17, 100,000 cells were isolated and their RNA extracted using the trizol method as previously described (Nicolaides N.C., Kinzler, K.W., and Vogelstein, B. (1995) Analysis of the 5' region of PMS2 reveals heterogeneous transcripts and a novel overlapping gene. Genomics 29:329-334). RNAs were reverse transcribed using Superscript II (Life Technologies) and PCR amplified using a sense primer located in exon 1 of the EF gene (5'-ttt cgc aac ggg ttt gcc g-3') (SEQ ID NO:17) and an antisense primer (5'-gtt tca gag tta agc ctt cg-3') (SEQ ID NO:18) centered at nt 283 of the published human PMS2 cDNA, which will detect both the full length as well as the PMS134 gene expression. Reactions were carried out using buffers and conditions as previously described (Nicolaides, N.C., et al. (1995) Genomic organization of the human PMS2 gene family. Genomics 30:195-206), using the following amplification parameters: 94°C for 30 sec, 52°C for 2 min, 72°C for 2 min, for 30 cycles. Reactions were analyzed on agarose gels. Figure 1 shows a representative example of PMS expression in stably transduced H36 cells.

Expression of the protein encoded by these genes were confirmed via western blot using a polyclonal antibody directed to the first 20 amino acids located in the N-terminus of the protein following the procedures previously described (data not shown) (Nicolaides et al. (1998) A Naturally Occurring hPMS2 Mutation Can Confer a Dominant Negative Mutator Phenotype. Mol. Cell. Biol. 18:1635-1641.

### EXAMPLE 2: hPMS134 Causes a Defect in MMR Activity and hypermutability in hybridoma cells

A hallmark of MMR deficiency is the generation of unstable microsatellite repeats in the genome of host cells. This phenotype is referred to as microsatellite instability (MI) (Modrich, P. (1994) Mismatch repair, genetic stability, and cancer Science 266:1959-1960; Palombo, F., et al. (1994) Mismatch repair and cancer Nature 36:417). MI consists of deletions and/or insertions within repetitive mono-, di- and/or tri nucleotide repetitive sequences throughout the entire genome of a host cell. Extensive genetic analysis eukaryotic cells have found that the only biochemical defect that is capable of producing MI is defective MMR (Strand, M., et al. (1993) Destabilization of tracts of simple repetitive DNA in yeast by mutations affecting DNA mismatch repair Nature 365:274-276; Perucho, M. (1996) Cancer of the micro satellite mutator phenotype. Biol Chem. 377:675-684; Eshleman J.R., and Markowitz, S.D. (1996) Mismatch repair defects in human carcinogenesis. Hum. Mol. Genet. 5:1489-494). In light of this unique feature that defective MMR has on promoting MI, it is now used as a biochemical marker to survey for lack of MMR activity within host cells (Perucho, M. (1996) Cancer of the microsatellite mutator phenotype. Biol Chem. 377:675-684; Eshleman J.R., and Markowitz, S.D. (1996) Mismatch repair defects in human carcinogenesis. Hum. Mol. Genet. 5:1489-494; Liu, T., et al. (2000) Microsatellite instability as a predictor of a mutation in a DNA mismatch repair gene in familial colorectal cancer Genes Chromosomes Cancer 27:17-25).

A method used to detect MMR deficiency in eukaryotic cells is to employ a reporter gene that has a polynucleotide repeat inserted within the coding region that disrupts its reading frame due to a frame shift. In the case where MMR is defective, the reporter gene will acquire random mutations (i.e. insertions and/or deletions) within the polynucleotide repeat yielding clones that contain a reporter with an open reading frame. We have employed the use of an MMR-sensitive reporter gene to measure for MMR activity in HBvec, HBPMS2, and HBPMS134 cells. The reporter construct used the pCAR-OF, which contains a hygromycin resistance (HYG) gene plus a β-galactosidase gene containing a 29 bp out-of-frame poly-CA tract at the 5' end of its coding region. The pCAR-OF reporter would not generate β -galactosidase activity unless a frame-restoring mutation (*i*.*e*., insertion or deletion) arose following transfection. HBvec, HBPMS2, and HB134 cells were each transfected with pCAR-OF vector in duplicate reactions following the protocol described in Example 1. Cells were selected in 0.5 mg/ml G418 and 0.5mg/ml HYG to select for cells retaining both the MMR effector and the pCAR-OF reporter plasmids. All cultures transfected with the pCAR vector resulted in a similar number of HYG/G418 resistant cells. Cultures were then expanded and tested for β-galactosidase activity *in situ* as well as by biochemical analysis of cell extracts. For *in situ* analysis, 100,000 cells were harvested and fixed in 1% gluteraldehyde, washed in phosphate buffered saline solution and incubated in 1 ml of X-gal substrate solution [0.15 M NaCl, 1 mM MgCl₂, 3.3 mM K₄Fe(CN)₆, 3.3 mM K₃Fe(CN)₆, 0.2% X-Gal ] in 24 well plates for 2 hours at 37°C. Reactions were stopped in 500 mM sodium bicarbonate solution and transferred to microscope slides for analysis. Three fields of 200 cells each were counted for blue (β-galactosidase positive cells) or white (β-galactosidase negative cells) to assess for MMR inactivation. Table 1 shows the results from these studies. While no β-galactosidase positive cells were observed in HBvec cells, 10% of the cells per field were β-galactosidase positive in HB134 cultures and 2% of the cells per field were β-galactosidase positive in HBPMS2 cultures.

Cell extracts were prepared from the above cultures to measure β-galactosidase using a quantitative biochemical assay as previously described (Nicolaides et al. (1998) A Naturally Occurring hPMS2 Mutation Can Confer a Dominant Negative Mutator Phenotype Mol. Cell. Biol. 18:1635-1641; Nicolaides, N.C., et al. (1992) The Jun family members, c-JUN and JUND, transactivate the human c-myb promoter via an Ap1 like element. J. Biol. Chem. 267:19665-19672). Briefly, 100,000 cells were collected, centrifuged and resuspended in 200 µls of 0.25M Tris, pH 8.0. Cells were lysed by freeze/thawing three times and supernatants collected after microfugation at 14,000 rpms to remove cell debris. Protein content was determined by spectrophotometric analysis at OD²⁸⁰. For biochemical assays, 20 µg of protein was added to buffer containing 45 mM 2-mercaptoethanol, 1mM MgCl₂, 0.1 M NaPO₄ and 0.6 mg/ml Chlorophenol red-β-D-galactopyranoside (CPRG, Boehringer Mannheim). Reactions were incubated for 1 hour, terminated by the addition of 0.5 M Na₂CO₃, and analyzed by spectrophotometry at 576 nm. H36 cell lysates were used to subtract out background. Figure 2 shows the β-galactosidase activity in extracts from the various cell lines. As shown, the HB134 cells produced the highest amount of β-galactosidase, while no activity was found in the HBvec cells containing the pCAR-OF. These data demonstrate the ability to generate MMR defective hybridoma cells using dominant negative MMR gene alleles.

**Table 1.** β-galactosidase expression of HBvec, HBPMS2 and HB134 cells transfected with pCAR-OF reporter vectors. Cells were transfected with the pCAR-OF β-galactosidase reporter plasmid. Transfected cells were selected in hygromycin and G418, expanded and stained with X-gal solution to measure for β-galactosidase activity (blue colored cells). 3 fields of 200 cells each were analyzed by microscopy. The results below represent the mean +/- standard deviation of these experiments. produced by each clone. Reactions are stopped by adding 50 µls of 500mM sodium bicarbonate and analyzed by OD at 415nm using a BioRad plate reader. Clones exhibiting an enhanced signal over background cells (H36 control cells) are then isolated and expanded into 10 ml cultures for additional characterization and confirmation of ELISA data in triplicate experiments. ELISAs are also performed on conditioned (CM) from the same clones to measure total Ig production within the conditioned medium of each well. Clones that produce an increased ELISA signal and have increased antibody levels are then further analyzed for variants that over-express and/or over-secrete antibodies as described in Example 4. Analysis of five 96-well plates each from HBvec or HB134 cells have found that a significant number of clones with a higher Optical Density (OD) value is observed in the MMR-defective HB134 cells as compared to the HBvec controls. Figure 4 shows a representative example of HB134 clones producing antibodies that bind to specific antigen (in this case IgE) with a higher affinity. Figure 4 provides raw data from the analysis of 96 wells of HBvec (left graph) or HB134 (right graph) which shows 2 clones from the HB134 plate to have a higher OD reading due to 1) genetic alteration of the antibody variable domain that leads to an increased binding to IgE antigen, or 2) genetic alteration of a cell host that leads to over-production/secretion of the antibody molecule. Anti-Ig ELISA found thatthe two clones, shown in figure 4 have Ig levels within their CM similar to the surrounding wells exhibiting ower OD values. These data suggest that a genetic alteration occurred within the antigen binding domain of the antibody which in turn allows for higher binding to antigen.

Clones that produced higher OD values as determined by ELISA were further analyzed at the genetic level to confirm that mutations within the light or heavy chain variable region have occurred that lead to a higher binding affinity hence yielding to a stronger ELISA signal. Briefly, 100,000 cells are harvested and extracted for RNA using the Triazol method as described above. RNAs are reverse transcribed using Superscript II as suggested by the manufacturer (Life Technology) and PCR amplified for the antigen binding sites contained within the variable light and heavy chains. Because of the heterogeneous nature of these genes, the following degenerate primers are used to amplify light and heavy chain alleles from the parent H36 strain.
Light chain sense: 5'-GGA TTT TCA GGT GCA GAT TTT CAG-3' (SEQ ID NO:1)
Light chain antisense: 5'-ACT GGA TGG TGG GAA GAT GGA-3' (SEQ ID NO:2)
Heavy chain sense: 5'-A(G/T) GTN (A/C)AG CTN CAG (C/G)AG TC-3' (SEQ ID NO:3)
Heavy chain antisense: 5'-TNC CTT G(A/G)C CCC AGT A(G/A)(A/T)C-3' (SEQ ID NO:4)

PCR reactions using degenerate oligonucleotides are carried out at 94°C for 30 sec, 52°C for 1 min, and 72°C for 1 min for 35 cycles. Products are analyzed on agarose gels. Products of the expected molecular weights are purified from the gels by Gene Clean (Bio 101), cloned into T-tailed vectors, and sequenced to identify the wild type sequence of the variable light and heavy chains. Once the wild type sequence has been determined, nondegenerate primers were made for RT-PCR amplification of positive HB134 clones. Both the light and heavy chains were amplified, gel purified and sequenced using the corresponding sense and antisense primers. The sequencing of RT-PCR products gives representative sequence data of the endogenous immunoglobulin gene and not due to PCR induced mutations. Sequences from clones were then compared to the wild type sequence for sequence comparison. An example of the ability to create *in vivo* mutations within an immunoglobulin light or heavy chain is shown in figure 5, where HB134 clone92 was identified by ELISA to have an increased signal for hIgE. The light chain was amplified using specific sense and antisense primers. The light chain was RT-PCR amplified and the resulting product was purified and analyzed on an automated ABI377 sequencer. As shown in clone A, a residue -4 upstream of the CDR region 3 had a genetic change from ACT to TCT, which results in a Thr to Ser change within the framework region just preceding the CDR#3. In clone B, a residue -6 upstream of the CDR region had a genetic change from CCC to CTC, which results in a Pro to Leu change within framework region proceding CDR#2.

The ability to generate random mutations in immunoglobulin genes or chimeric immunoglobulin genes is not limited to hybridomas. Nicolaides et al. (Nicolaides et al. (1998) A Naturally Occurring hPMS2 Mutation Can Confer a Dominant Negative Mutator Phenotype Mol. Cell. Biol. 18:1635-1641) has previously shown the ability to generate hypermutable hamster cells and produce mutations within an endogenous gene. A common method for producing humanized antibodies is to graft CDR sequences from a MAb (produced by immunizing a rodent host) onto a human Ig backbone, and transfection of the chimeric genes into Chinese Hamster Ovary (CHO) cells whih in turn produce a functional Ab that is secreted by the CHO cells (Shields, R.L., et al. (1995) Anti-IgE monoclonal antibodies that inhibit allergen-specific histamine release. Int. Arch Allergy Immunol. 107:412-413). The methods described within this application are also useful for generating genetic alterations within Ig genes or chimeric Igs transfected within host cells such as rodent cell lines, plants, yeast and prokaryotes (Frigerio L, et al. (2000) Assembly, secretion, and vacuolar delivery of a hybrid immunoglobulin in plants. Plant Physiol. 123:1483-1494).

These data demonstrate the ability to generate hypermutable hybridomas, or other Ig producing host cells that can be grown and selected, to identify structurally altered immunoglobulins yielding antibodies with enhanced biochemical properties, including but not limited to increased antigen binding affinity. Moreover, hypermutable clones that contain missense mutations within the immunoglobulin gene that result in an amino acid change or changes can be then further characterized for *in vivo* stability, antigen clearance, on-off binding to antigens, etc. Clones can also be further expanded for subsequent rounds of *in vivo* mutations and can be screened using the strategy listed above.

The use of chemical mutagens to produce genetic mutations in cells or whole organisms are limited due to the toxic effects that these agents have on "normal" cells. The use of chemical mutagens such as MNU in MMR defective organisms is much more tolerable yielding to a 10 to 100 fold increase in genetic mutation over MMR deficiency alone (Bignami M, (2000) Unmasking a killer: DNA O(6)-methylguanine and the cytotoxicity of methylating agents. Mutat. Res. 462:71-82). This strategy allows for the use of chemical mutagens to be used in MMR-defective Ab producing cells as a method for increasing additional mutations within immunoglobulin genes or chimeras that may yield functional Abs with altered biochemical properties such as enhanced binding affinity to antigen, etc.

### Example 4: Generation of antibody producing cells with enhanced antibody production

Analysis of clones from H36 and HB 134 following the screening strategy listed above hasidentified a significant number of clones that produce enhanced amounts of antibody into the medium. While a subset of these clones gave higher Ig binding data as determined by ELISA as a consequence of mutations within the antigen binding domains contained in the variable regions, others were found to contain "enhanced" antibody production. A summary of the clones producing enhanced amounts of secreted MAb is shown in TABLE 2, where a significant number of clones from HB 134 cells were found to produce enhanced Ab production within the conditioned medium as compared to H36 control cells.

**TABLE 2.** Generation of hybridoma cells producing high levels of antibody. HB 134 clones were assayed by ELISA for elevated Ig levels. Analysis of 480 clones showed that a significant number of clones had elevated MAb product levels in their CM. Quantification showed that several of these clones produced greater than 500ngs/ml of MAb due to either enhanced expression and/or secretion as compared to clones from the H36 cell line.

**Table 2. Production of MAb in CM from H36 and HB134 clones.**

| Cell Line | % clones > 400 ng/ml | % clones >500 ng/ml |
|---|---|---|
| H36 | 1/480 = 0.2% | 0/480 = 0% |
| HB 134 | 50/480 = 10% | 8/480 = 1.7% |

Cellular analysis of HB 134 clones with higher MAb levels within the conditioned medium (CM) were analyzed to determine if the increased production was simply due to genetic alterations at the Ig locus that may lead to over-expression of the polypeptides forming the antibody, or due to enhanced secretion due to a genetic alteration affecting secretory pathway mechanisms. To address this issue, we expanded three HB 134 clones that had increased levels of antibody within their CM. 10,000 cells were prepared for western blot analysis to assay for intracellular steady state Ig protein levels (Figure 6). In addition, H36 cells were used as a standard reference (Lane 2) and a rodent fibroblast (Lane 1) was used as an Ig negative control. Briefly, cells were pelleted by centrifugation and lysed directly in 300 µl of SDS lysis buffer (60 mM Tris, pH 6.8, 2% SDS, 10% glycerol, 0.1 M 2-mercaptoethanol, 0.001% bromophenol blue) and boiled for 5 minutes. Lysate proteins were separated by electrophoresis on 4-12% NuPAGE gels (for analysis of Ig heavy chain. Gels were electroblotted onto Immobilon-P (Millipore) in 48 mM Tris base, 40 mM glycine, 0.0375% SDS, 20% methanol and blocked at room temperature for 1 hour in Tris-buffered saline (TBS) plus 0.05% Tween-20 and 5% condensed milk. Filters were probed with a 1:10,000 dilution of sheep anti-mouse horseradish peroxidase conjugated monoclonal antibody in TBS buffer and detected by chemiluminescence using Supersignal substrate (Pierce). Experiments were repeated in duplicates to ensure reproducibility. Figure 6 shows a representative analysis where a subset of clones had enhanced Ig production which accounted for increased Ab production (Lane 5) while others had a similar steady state level as the control sample, yet had higher levels of Ab within the CM. These data suggest a mechanism whereby a subset of HB134 clones contained a genetic alteration that in turn produces elevated secretion of antibody.

The use of chemical mutagens to produce genetic mutations in cells or whole organisms are limited due to the toxic effects that these agents have on "normal" cells. The use of chemical mutagens such as MNU in MMR defective organisms is much more tolerable yielding to a 10 to 100 fold increase in genetic mutation over MMR deficiency alone (Bignami M, (2000) Unmasking a killer: DNA O(6)-methylguanine and the cytotoxicity of methylating agents. Mutat. Res. 462:71-82). This strategy allows for the use of chemical mutagens to be used in MMR-defective Ab producing cells as a method for increasing additional mutations within immunoglobulin genes or chimeras that may yield functional Abs with altered biochemical properties such as enhanced binding affinity to antigen, etc.

### Example 5: establishment of genetic stability in hybridoma cells with new output trait.

The initial steps of MMR are dependent on two protein complexes, called MutSα and MutLα (Nicolaides et al. (1998) A Naturally Occurring hPMS2 Mutation Can Confer a Dominant Negative Mutator Phenotype. Mol. Cell. Biol. 18:1635-1641). Dominant negative MMR alleles are able to perturb the formation of these complexes with downstream biochemicals involved in the excision and polymerization of nucleotides comprising the "corrected" nucleotides. Examples from this application show the ability of a truncated MMR allele (PMS 134) as well as a full length human PMS2 when expressed in a hybridoma cell line is capable of blocking MMR resulting in a hypermutable cell line that gains genetic alterations throughout its entire genome per cell division. Once a cell line is produced that contains genetic alterations within genes encoding for an antibody, a single chain antibody, over expression of immunoglobulin genes and/or enhanced secretion of antibody, it is desirable to restore the genomic integrity of the cell host. This can be achieved by the use of inducible vectors whereby dominant negative MMR genes are cloned into such vectors, introduced into Ab producing cells and the cells are cultured in the presence of inducer molecules and/or conditions. Inducible vectors include but are not limited to chemical regulated promoters such as the steroid inducible MMTV, tetracycline regulated promoters, temperature sensitive MMR gene alleles, and temperature sensitive promoters.

The results described above lead to several conclusions. First, expression of hPMS2 and PMS134 results in an increase in microsatellite instability in hybridoma cells. That this elevated microsatellite instability is due to MMR deficiency was proven by evaluation of extracts from stably transduced cells. The expression of PMS 134 results in a polar defect in MMR, which was only, observed using heteroduplexes designed to test repair from the 5' direction (no significant defect in repair from the 3' direction was observed in the same extracts) (Nicolaides et al. (1998) A Naturally Occurring hPMS2 Mutation Can Confer a Dominant Negative Mutator Phenotype. Mol. Cell. Biol. 18:1635-1641), Interestingly, cells deficient in hMLH1 also have a polar defect in MMR, but in this case preferentially affecting repair from the 3' direction (Drummond, J.T, et al. (1996) Cisplatin and adriamycin resistance are associated with MutLa and mismatch repair deficiency in an ovarian tumor cell line. J. Biol. Chem. 271:9645-19648).

Another method that is taught in this application is that ANY method used to block MMR using *PMS2* can be performed to generate hypermutablility in an antibody-producing cell that can lead to genetically altered antibodies with enhanced biochemical features such as but not limited to increased antigen binding, enhanced pharmacokinetic profiles, etc. These processes can also to be used to generate antibody producer cells that have increased Ig expression as shown in Example 4, figure 6 and/or increased antibody secretion as shown in Table 2.

In addition, we demonstrate the utility of blocking MMR using *PMS2* in antibody producing cells to increase genetic alterations within Ig genes that may lead to altered biochemical features such as, but not limited to, increased antigen binding affinities (Figure 5A and 5B). The blockade of MMR in such cells can be through the use of dominant negative MMR *PMS2* from any species including bacteria, yeast, protozoa, insects, rodents, primates, mammalian cells, and man.

## Claims

1. A method of making a hypermutable, antibody-producing cell, comprising introducing into a cell capable of producing antibodies a polynucleotide comprising a dominant negative allele of a mismatch repair gene, wherein said mismatch repair gene is PMS2.

2. A method according to claim 1, wherein said capability is due to the co-introduction of an immunoglobulin gene into said cell.

3. A method according to claim 1, comprising the step of restoring the genetic stability of said hypermutable cell.

4. A method of generating a mutation in an antibody-producing cell, the mutation causing over expression, enhanced secretion or enhanced affinity for antigen of an antibody produced by the cell, the method comprising:
growing said antibody-producing cell comprising a dominant negative allele of a mismatch repair gene; and
testing for the over expression, enhanced secretion or enhanced affinity for antigen of an antibody produced by the cell,
wherein said mismatch repair gene is PMS2.

5. A method of reversibly altering the hypermutability of an antibody-producing cell, comprising introducing an inducible vector into said cell, wherein said inducible vector comprises a dominant negative allele of a mismatch repair gene operably linked to an inducible promoter, and inducing said cell to express said dominant negative mismatch repair gene; wherein said mismatch repair gene is PMS2.

6. A method according to claim 5, comprising ceasing induction of said cell, thereby restoring the genetic stability of said cell.

7. A method of producing genetically altered antibodies, comprising
transfecting a polynucleotide encoding an immunoglobulin protein into a cell, wherein said cell comprises a dominant negative allele of a mismatch repair gene;
growing said cell, thereby producing a hypermutated polynucleotide encoding a hypermutated immunoglobulin protein;
screening for a desirable property of said hypermutated immunoglobulin protein;
isolating said hypermutated polynucleotide; and
transfecting said hypermutated polynucleotide into a genetically stable cell, thereby producing a hypermutated antibody-producing, genetically stable cell;
wherein said mismatch repair gene is PMS2.

8. A method according to any preceding claim, wherein said dominant negative allele comprises a truncation mutation.

9. A method according to claim 8, wherein said dominant negative allele encodes a protein consisting of the first 133 amino acids of hPMS2.

10. A hypermutated antibody-producing cell comprising a polynucleotide encoding an antibody and a polynucleotide encoding a dominant negative allele of PMS2.

11. A hypermutated antibody-producing cell as claimed in claim 10, wherein said cell is genetically stable.

12. A homogeneous culture of the cells of claim 10 or claim 11.

## Patentansprüche

1. Verfahren zur Herstellung einer hypermutationsfähigen Antikörper produzierenden Zelle, wobei in eine zur Herstellung von Antikörpern geeignete Zelle ein Polynukleotid eingeführt wird welches einen dominant negativen Erbgutfaktor eines unpassenden Reparaturgens enthält, wobei gesagtes unpassendes Reparaturgen PMS2 ist.

2. Verfahren nach Anspruch 1, wobei besagte Befähigung abhängig von der Co-Einführung eines immunglobulingens in besagte Zelle ist.

3. Verfahren nach Anspruch 1, enthaltend den Schritt der Wiederherstellung der genetischen Stabilität besagter hypermutationsfähiger Zelle.

4. Verfahren zur Erzeugung einer Mutation in einer Antikörper produzierenden Zelle, wobei die Mutation über Expression verstärkte Sekretion oder verstärkte Affinität für ein Antigen eines Antikörpers, hergestellt durch die Zelle, veranlasst, und die Methode beinhaltet: Wachstum besagter Antikörper produzierender Zelle enthaltend einen dominant negativen Erbgutfaktor eines unpassenden Reparaturgens und für die Überexpression Prüfung der verstärkten Sekretion oder der verstärkten Affinität für Antigene eines Antikörpers, hergestellt durch die Zelle, wobei besagtes unpassendes Reparaturgen PMS2 ist.

5. Verfahren zur reversiblen Veränderung der Hypermutierfähigkeit einer Antikörper produzierenden Zelle, beinhaltend die Einführung eines einführbaren Vektors in besagte Zelle, wobei besagter einführbarer Vektor einen dominant negativen Erbgutfaktor eines unpassenden Reparaturgens enthält, der an einen einführbaren Promoter operativ angehängt ist und Einführen besagter Zelle zur Expression besagten dominant negativen unpassenden Reparaturgens, wobei besagtes unpassendes Reparaturgen PMS2 ist.

6. Verfahren nach Anspruch 5 enthaltend Unterlassung der Induktion besagter Zelle um auf diese Weise die genetische Stabilität besagter Zelle wieder herzustellen.

7. Verfahren zur Herstellung genetisch veränderter Antikörper enthaltend die Übertragung einer polynukleotiden Kodierung eines Immunglobulinproteins in eine Zelle, wobei besagte Zelle ein dominant negatives Erbgut eines nicht passenden Reparaturgens enthält; Wachstum besagter Zelle um auf diese Weise ein hypermutiertes Polynukleotid zu erzeugen, welches ein hypermutiertes Immunglobulinprotein kodiert; suchen einer gewünschten Eigenschaft besagten hypermutierten Immunglobulinproteins; Isolieren besagten hypermutierten Polynukleotids und Übertragung besagten hypermutierten Polynukleotids in eine genetisch stabile Zelle, um auf diese Weise eine genetisch stabile hypermutierte Antikörper herstellende Zelle zu erstellen, wobei besagtes unpassendes Reparaturgen PMS2 ist.

8. Verfahren nach einem der vorangegangenen Ansprüche, wobei besagter dominant negativer Erbgutfaktor eine verstümmelte Mutation enthält.

9. Verfahren nach Anspruch 8, wobei besagter dominant negativer Erbgutfaktor ein Protein kodiert welches aus den ersten 133 Aminosäuren von hPMS2 besteht.

10. Eine hypermutierte Antikörper produzierende Zelle enthaltend ein Polynukleotid kodierend einen Antikörper und ein Polynukleotid kodierend einen dominant negativen Erbgutfaktor von PMS2.

11. Eine hypermutierte Antikörper produzierende Zelle nach Anspruch 10, wobei besagte Zelle genetisch stabil ist.

12. Eine homogene Kultur der Zellen nach Anspruch 10 oder 11.

## Revendications

1. Procédé pour préparer une cellule hypermutable produisant des anticorps, comprenant l'introduction dans une cellule capable de produire des anticorps d'un polynucléotide comprenant un allèle négatif dominant d'un gène de réparation des appariements, dans lequel ledit gène de réparation des appariements est le PMS2.

2. Procédé selon la revendication 1, dans lequel ladite capacité est due à la co-introduction d'un gène d'immunoglobuline dans ladite cellule.

3. Procédé selon la revendication 1, comprenant l'étape consistant à restaurer la stabilité génétique de ladite cellule hypermutable.

4. Procédé pour générer une mutation dans une cellule produisant des anticorps, la mutation provoquant la surexpression, l'amélioration de la sécrétion ou l'amélioration de l'affinité pour l'antigène d'un anticorps produit par la cellule, le procédé comprenant :
la culture de ladite cellule produisant des anticorps comprenant un allèle négatif dominant d'un gène de réparation des appariements ; et
le test de la surexpression, de la sécrétion améliorée ou de l'affinité améliorée pour l'antigène d'un anticorps produit par la cellule,
dans lequel ledit gène de réparation des appariements est le PMS2.

5. Procédé pour modifier de manière réversible la capacité d'hypermutation d'une cellule produisant des anticorps, comprenant l'introduction d'un vecteur inductible dans ladite cellule, dans lequel ledit vecteur inductible comprend un allèle négatif dominant d'un gène de réparation des appariements lié de manière opérationnelle à un promoteur inductible, et l'induction de ladite cellule pour exprimer ledit gène de réparation des appariements négatif dominant ; dans lequel ledit gène de réparation des appariements est le PMS2.

6. Procédé selon la revendication 5, comprenant la cessation de l'induction de ladite cellule, pour ainsi restaurer la stabilité génétique de ladite cellule.

7. Procédé pour produire des anticorps génétiquement modifiés, comprenant
la transfection d'un polynucléotide codant une protéine d'immunoglobuline dans une cellule, ladite cellule comprend un allèle négatif dominant d'un gène de réparation des appariements ;
la culture de ladite cellule, pour ainsi produire un polynucléotide hypermuté codant une protéine d'immunoglobuline hypermutée ;
le criblage d'une propriété souhaitée de ladite protéine d'immunoglobuline hypermutée ;
l'isolement dudit polynucléotide hypermuté ; et
la transfection dudit polynucléotide hypermuté dans une cellule génétiquement stable, pour ainsi produire une cellule génétiquement stable produisant des anticorps hypermutés ;
dans lequel ledit gène de réparation des appariements est le PMS2.

8. Procédé selon l'une quelconque des revendications précédentes, dans lequel ledit allèle négatif dominant comprend une mutation par troncature.

9. Procédé selon la revendication 8, dans lequel ledit allèle négatif dominant code une protéine constituée par les 133 premiers acides aminés de hPMS2.

10. Cellule produisant des anticorps hypermutés comprenant un polynucléotide codant un anticorps et un polynucléotide codant un allèle négatif dominant de PMS2.

11. Cellule produisant des anticorps hypermutés selon la revendication 10, dans laquelle ladite cellule est génétiquement stable.

12. Culture homogène des cellules selon la revendication 10 ou la revendication 11.
